# EUROPÄISCHE PATENTSCHRIFT

(11) **EP 3 723 603 B1**
(45) Veröffentlichungstag und Bekanntmachung des Hinweises auf die Patenterteilung: **28.09.2022**
(21) Anmeldenummer: 18829224.7
(22) Anmeldetag: 07.12.2018
(51) Int. Cl.: A61B 5/103, A61B 5/00, G01N 21/359, G01N 21/64, G01N 21/25, G01N 21/3563, A45D 44/00, G01N 21/3554

(54) **HAARZUSTANDS-ERMITTLUNGSVORRICHTUNG UND VERFAHREN ZUM BEREITSTELLEN VON HAARZUSTANDSINFORMATIONEN**
HAIR CONDITION DETERMINING DEVICE AND METHOD FOR PROVIDING HAIR CONDITION INFORMATION
DISPOSITIF DE DÉTERMINATION D'ÉTAT D'UNE CHEVELURE ET PROCÉDÉ POUR FOURNIR DES INFORMATIONS D'ÉTAT DE CHEVELURE

(30) Priorität: 11.12.2017 DE 102017222421
(43) Veröffentlichungstag der Anmeldung: 21.10.2020
(73) Patentinhaber: Henkel AG & Co. KGaA, 40589 Düsseldorf (DE)
(72) Erfinder: KATZAROV, Jordan, 40591 Düsseldorf (DE); LEHANNE, Adrian, 10179 Berlin (DE)
(86) Internationale Anmeldenummer: PCT/EP2018/083912
(87) Internationale Veröffentlichungsnummer: WO 2019/115370

(56) Entgegenhaltungen:
- WO-A1-2017/198479
- JP-A- 2004 198 398
- JP-B2- 3 641 370
- US-A1- 2015 342 515
- US-A1- 2017 164 887
- US-B1- 7 443 508

## Beschreibung

Die Erfindung betrifft eine Haarzustands-Ermittlungsvorrichtung nach Anspruch 1 und ein Verfahren zum Bereitstellen von Haarzustandsinformationen nach Anspruch 13.

In vielen Bereichen des täglichen Lebens gibt es seit einiger Zeit einen Trend zu personalisierten Programmen, die auf individuelle Voraussetzungen und Bedürfnisse gezielt eingehen können, beispielsweise in einem Ernährungs- oder Gesundheitsbereich, aber auch in einem Bereich personalisierter Kosmetik. Diese kann es einem Nutzer ermöglichen, gezielt Kosmetikprodukte zu finden und/oder Pflegehinweise zu erhalten, die auf individuelle Bedürfnisse seiner Haare abgestimmt sind, und somit eine besonders hohe Wirksamkeit ermöglichen.

Insbesondere haben junge Friseure und Friseurinnen noch nicht die fundierte Erfahrung im Umgang mit geschädigtem Haar, beispielsweise welches Pflegeprodukt oder welche Kombination aus Pflegeprodukten bei einer Behandlung von geschädigtem Haar zielführend ist. Insbesondere braucht es auch sehr viel Erfahrung, um bei geschädigtem Haar ein erfolgreiches Haarfärbeerlebnis zu erreichen und um zu wissen wie Haarfärbeprodukte zusammengemischt werden müssen, um selbst bei geschädigtem Haar das gewünschte Färbeergebnis zu erreichen.

Bei einer Behandlung von Haar mit kosmetischen Produkten kann eine Wirkung des Produkts, z.B. eine Intensität einer Färbung, eine Wirksamkeit eines Pflegeprodukts oder eine Haarumformungswirkung einer Dauerwelle, stark von einem Haarzustand, insbesondere einem Schädigungsgrad des Haars abhängen.

Deshalb kann eine präzise Ermittlung einer Haarfarbe und einer Schädigung des Haars von großer Bedeutung sein und für den Nutzer wichtige Parameter zur (objektiven) Beurteilung seiner Haargesundheit darstellen.

Für viele Menschen, die sich ein gesundes und gepflegtes Haar wünschen, stellt der Feuchtigkeitsgehalt ihrer Haare einen wichtigen Haarparameter dar.

Ferner besteht Bedarf an einer Vorrichtung zum Erfassen von Haarzustandsinformation, die eine zerstörungsfreie Messung insbesondere am Lebendhaar einer Person ermöglicht.

Ferner besteht Bedarf an einer Vorrichtung zum Erfassen von Haarzustandsinformation, die intuitiv mit einer Hand eines Nutzers steuerbar ist und dem Nutzer ein Erfassen der Haarzustandsinformation mit der Vorrichtung vereinfacht.

Ferner besteht Bedarf an einer Vorrichtung zum Erfassen von Haarzustandsinformation, die eine einfache und schnelle Kalibrierung mindestens eines Sensors, der zur Erfassung von Haarzustandsinformation eingesetzt wird, erlaubt.

Die Schädigung des Haars kann durch natürliche oder künstlich herbeigeführte Vorgänge geschehen. Der wichtigste Schädigungstyp kann dabei eine oxidative Schädigung sein.

Die natürlichen Vorgänge können beispielsweise eine kombinierte (z.B. gleichzeitige) Einwirkung von UV-Licht und Sauerstoff (O2) auf das Haar aufweisen.

Die künstlich herbeigeführten Vorgänge können dabei beispielsweise ein Anwenden von Haarfärbemitteln (auch als Colorationen bezeichnet, wozu hierin auch ein Blondieren gezählt wird), und/oder ein Stylen bzw. Umformen des Haars (z.B. ein Erzeugen einer Dauerwelle) aufweisen. Dabei kann neben erwünschten kosmetischen Effekten, wie z.B. einer Aufhellung des Haars, auch eine starke Schädigung des Haars auftreten, beispielsweise bei einer Verwendung von Oxidationsmitteln.

Beim geschädigten Haar kann beispielsweise ein Cysteinsäuregehalt erhöht sein wegen einer Oxidation der im Haar sehr häufig vorkommenden Aminosäuren Cystin und Cystein zu Cysteinsäure.

Die Oxidation des Cystins/Cysteins zu Cysteinsäure kann die mechanische Stabilität des Haars zerstören und bei mehrfachen Anwendungen sogar zu einem vollständigen Haarbruch führen. Allerdings können bereits vorher makroskopisch wahrnehmbare, z.B. fühlbare, Eigenschaften des Haars, beispielsweise eine Oberflächenbeschaffenheit, z.B. eine Oberflächenrauhigkeit, negativ beeinflusst werden. Geschädigtes Haar kann beispielsweise eine höhere Oberflächenrauhigkeit aufweisen als ungeschädigtes Haar.

Ergebnisse kosmetischer Behandlungen können von weiteren Eigenschaften des behandelten Haars abhängen, beispielsweise von einer Haarfarbe (insbesondere bei einer Coloration), von einer Haarstruktur (insbesondere bei einem Styling, z.B. einer Dauerwelle, einer Glättung usw.), von einem Feuchtigkeitsgehalt (bei einem Pflegeprodukt), usw.

Dokument JP3641370 B2 beschreibt eine Vorrichtung zur Messung des Haarglanzes, die auf das Haar geklippt werden kann. Dokument US20170164887 A1 beschreibt eine Vorrichtung, die Haareigenschaften, wie zum Beispiel die Haarfeuchtigkeit innerhalb eines Helms oder einer Haarklammer, messen kann. Dokument JP2004198398A beschreibt einen Farbsensor, der zur Bestimmung der Haarfarbe konfiguriert ist. Dokument US20150342515A1 beschreibt eine angeschlossene Haarbürste mit Beschleunigungssensoren, die in der Lage sind, Haarparameter zu bestimmen. US 7 443 508 B1 beschreibt einen Kalibrierstandard für Reflektionsmessungen. WO 2017/198479 A1 beschreibt die Bestimmung von Cysteinsäure in Haar mittels Fluoreszenzmessungen.

In verschiedenen Ausführungsbeispielen wird eine Haarzustands-Ermittlungsvorrichtung bereitgestellt, welche es ermöglicht, einen Haarzustand eines Nutzers zu ermitteln. Das Ermitteln des Haarzustands kann in verschiedenen Ausführungsbeispielen während eines Abstreifens an oder Aufnehmens von Haar mittels der Haarzustands-Ermittlungsvorrichtung erfolgen, d.h. die Haarzustands-Ermittlungsvorrichtung kann derart eingerichtet sein, dass eine Führungsmöglichkeit für Haar realisiert sein kann.

In verschiedenen Ausführungsbeispielen kann durch aufeinanderfolgende Messungen entlang einer Haarprobe eine Ermittlung des Zustands und der Entwicklung über eine Zeitachse vorgenommen werden, z.B. für die Feststellung eines Schädigungs- oder Veränderungsbereichs. In verschiedenen Ausführungsbeispielen kann anhand des ermittelten Haarzustands eine Empfehlung bereitgestellt werden, z.B. bezüglich eines kosmetischen Haarbehandlungsprodukts, einer Zusammensetzung eines Haarbehandlungsprodukts und/oder einer Haarbehandlungsempfehlung.

In verschiedenen Ausführungsbeispielen kann eine hierin beschriebene Haarzustands-Ermittlungsvorrichtung und ein Verfahren als Teil eines "Smart Salon" Systems vorgesehen sein, das ein ganzheitliches System umfassend eine Mehrzahl von intelligenten smarten Vorrichtungen ist, das eine Identifikation bzw. Analyse und eine Einschätzung bzw. Beratung hin zu einer Bereitstellung mindestens eines Haarbehandlungsproduktes zum Verbessern eines Haarzustands und/oder einer gewünschten Haarfarbe eines Nutzers bereitstellen kann.

Unter einem optischen Sensor ist hierin ein Sensor zu verstehen, welcher mittels optischer Elemente elektromagnetische Strahlung im Wellenbereich des sichtbaren und unsichtbaren Lichts, im weiteren Sinne kurzwellige Strahlung (UV-Licht), sichtbares Licht (auch abkürzend als VIS bezeichnet), langwellige Strahlung wie Nahinfrarotlicht (NIR) und/oder Infrarotlicht (IR)) leitet und mittels eines Detektors (z.B. eines elektronischen Detektors, z.B. für sichtbares Licht, für NIR- oder/und IR-Licht, eines Photometers oder Ähnlichem) erfasst, analysiert und weiterverarbeitet, insbesondere auch auf Basis der Spektroskopieverfahren für Infrarotstrahlen.

Hierin kann Bezug genommen werden auf "die Sensoren", beispielsweise hinsichtlich einer Datenübertragung zwischen den Sensoren und der Datenverarbeitungsvorrichtung, einer Anordnung der Sensoren, usw. Dies ist so zu verstehen, dass die Sensoren eine Gesamtheit von in bzw. an dem Vorrichtungskörper angeordneten Sensoren und/oder Sensorschaltkreisen aufweisen können, z.B. eine Gesamtheit von optischem/n Sensor/en (z.B. Spektrometer, Kamera, Mikroskop), Mikrofon/en, Geschwindigkeits-Sensorschaltkreis, usw., oder, sofern dies aus dem Kontext hervorgeht, einen Teil der genannten Sensoren und/oder Sensorschaltkreise.

Erfindungsgemäß ist einer der optischen Sensoren ein Nahinfrarotspektroskopie-Sensor (NIR-Sensor) , z.B. ein NIR-Spektrometer oder eine NIR-Kamera, welcher in weiteren Ausführungsbeispielen eingerichtet sein kann, neben der Haarschädigung auch einen Feuchtigkeitsgehalt zu ermitteln.

In verschiedenen Ausführungsbeispielen kann die Erfassung durch Absorption, Reflektion und Spektroskopie erfolgen, wobei die jeweiligen durchzuführenden Verfahren mittels einer mobilen bzw. portablen Datenverarbeitungsvorrichtung ausgewertet werden können und/oder mittels einer Netzwerksoftware (Cloud) ausgewertet werden können, um beispielsweise der Komplexität der Spektroskopieauswertung gerecht zu werden.

Die Haarzustands-Ermittlungsvorrichtung nach Anspruch 1 umfasst *inter alia* mindestens einen Nahinfrarotspektroskopie-Sensor, z.B. ein NIR-Spektrometer und/oder einen NIR/ VIS-Spektrometer, wie sie beispielsweise in den im Folgenden genannten Spektrometern verbaut sind.

In verschiedenen Ausführungsbeispielen kann beispielsweise als ein geeignetes Spektrometer das "MicroNIR OnSite" der Firma Viavi Solutions Inc. vorgesehen sein. In verschiedenen Ausführungsbeispielen kann das Spektrometer eine Messung mit einer Messdauer in einem Bereich von etwa 0,10 s (Sekunden) bis etwa 5 s, beispielsweise in einem Bereich von etwa 1 s bis etwa 3 s, beispielsweise von etwa 2 s, mindestens eine Aufnahme der Nahinfrarot- und/oder Infrarotspektren von Haar eines Verbrauchers annähernd in Echtzeit erfassen und/oder ermitteln. In verschiedenen Ausführungsbeispielen kann das Spektrometer mindestens eine integrierte Vakuum-Wolfram-Lampe und ein InGaAs-Photodioden-Array mit 128 Pixeln aufweisen. In verschiedenen Ausführungsbeispielen kann das "MicroNIR OnSite" in einem Wellenlängenbereich von etwa 6060 cm⁻¹ bis etwa 10526 cm⁻¹ arbeiten.

In verschiedenen Ausführungsbeispielen kann beispielsweise ein Spektrometer "i-Spec Nano" der Firma B&W Tek verwendet werden. In verschiedenen Ausführungsbeispielen kann das Spektrometer eine Lichtquelle aufweisen und in einem Wellenlängenbereich von etwa 4545 cm-1 bis etwa 7692 cm-1 arbeitet.

In verschiedenen Ausführungsbeispielen kann beispielsweise ein NIR/VIS-Spektrometer "QualitySpec Trek" der Firma ASD Inc. eingesetzt werden. In verschiedenen Ausführungsbeispielen kann das Spektrometer in einem Wellenlängenbereich von etwa 28571 cm⁻¹ bis etwa 400 cm⁻¹ (350 - 2500 nm) arbeiten.

In verschiedenen Ausführungsbeispielen kann beispielsweise ein Spektrometer das "SCiO by Consumer Physics" sein. Das Spektrometer kann im kurzwelligen Bereich des NIR und zwar bei Wellenlängen von etwa 9090 cm⁻¹ bis etwa 14285 cm⁻¹ (700 bis 1100 nm) arbeiten.

In verschiedenen Ausführungsbeispielen kann beispielsweise ein Spektrometer der Firma Attonics Systems eingesetzt werden, welches entweder in Wellenlängenbereichen von etwa 9090 cm⁻¹ bis etwa 26.315 cm⁻¹ (VIS-NIR) oder von etwa 3333 cm⁻¹ bis etwa 10.000 cm⁻¹ (NIR) arbeiten kann. In verschiedenen Ausführungsbeispielen kann das Spektrometer auf Interferometern basieren und einen hohen Lichtdurchsatz und eine hohe spektrale Auflösung (kleiner 5 nm für VIS-NIR-Spektrometer und größer 20 nm für das NIR-Spektrometer) aufweisen. In verschiedenen Ausführungsbeispielen kann das Spektrometer einen Multi-Phase-Shift-Array(MPA)-Chip und eine optische Anordnung in einem kreisförmigen Rohr aufweisen.

In verschiedenen Ausführungsbeispielen können beispielsweise für VIS-NIR-Spektrometer die Miniaturspektrometer "USB2000-VIS-NIR" oder "USB4000-VIS-NIR" der Firma Ocean Optics eingesetzt werden. In verschiedenen Ausführungsbeispielen kann das Spektrometer in einem Wellenlängenbereich von etwa 350 nm bis etwa 1000 nm arbeiten.

In verschiedenen Ausführungsbeispielen können als ein NIR-Auswertungsmodul die Module "DLP^{®} NIRscan" oder "DLP^{®} NIRscan Nano" der Firma Texas Instruments verwendet werden. In verschiedenen Ausführungsbeispielen weist das Auswertungsmodul zwei Wolfram-Lampen und InGaAs-Photodioden als Detektoren auf. In verschiedenen Ausführungsbeispielen kann das Modul "DLP^{®} NIRscan" in einem Wellenlängenbereich von etwa 4016 cm⁻¹ bis etwa 7407 cm⁻¹ und das Modul "DLP^{®} NIRscan Nano" in einem Bereich von etwa 5882 cm⁻¹ bis etwa 11111 cm⁻¹ arbeiten.

In verschiedenen Ausführungsbeispielen ist als ein weiterer geeigneter NIR-Sensor der "NeoSpectra" von Si-Ware Systems verwendbar, beispielsweise der Sensor NeoSpectra SW62221-1.7, der Sensor NeoSpectra SW62221-2.1 und der Sensor NeoSpectra SW62221-2.5, welche in unterschiedlichen Wellenlängenbereichen arbeiten können.

In verschiedenen Beispielen kann ein optischer Sensor beispielsweise ein Sensor für sichtbares Licht sein, z.B. ein Spektrometer oder eine Kamera für einen Spektralbereich des sichtbaren Lichts, insbesondere in einem Bereich, in welchem Fluoreszenzlicht von Haar emittiert wird. Dabei kann der optische Sensor eingerichtet sein, eine Haarschädigung zu ermitteln.

Die Vorrichtung nach Anspruch 1 umfasst *inter alia* einen optischen Sensor ausgebildet als Farbsensor zum Erfassen der Haarfarbe eines Nutzers. In einem Fall, dass die Kamera (oder eine zusätzliche Kamera) bereitgestellt ist, kann diese ferner genutzt werden, um eine Haardichte zu ermitteln.

Ein weiterer Sensor zum Ermitteln der Haarschädigung ist in der Haarzustands-Ermittlungsvorrichtung bereitgestellt wie in Anspruch 1 definiert. Eine Kombination von mehreren der oben genannten Sensoren ermöglicht, ein umfassenderes Bild des Haarzustands zu erhalten. Ein Erfassen von zwei oder mehr Haarzustandsparametern kann als zwei- bzw. mehrdimensionale Messung verstanden werden, mittels welcher ein Analyseergebnis bezüglich des Haarzustands verbessert werden kann.

In verschiedenen Ausführungsbeispielen kann die Haarzustands-Ermittlungsvorrichtung beim Ermitteln der Empfehlung, z.B. des Haarbehandlungsmittels, das Wunschergebnis berücksichtigen, derart, dass das empfohlene Produkt und/oder die empfohlene Haarbehandlung geeignet ist, am Haar des Nutzers das Wunschergebnis herbeizuführen.

In verschiedenen Ausführungsbeispielen kann als Eingabevorrichtung an der Haarzustands-Ermittlungsvorrichtung alternativ oder zusätzlich eine andere herkömmliche Eingabevorrichtung bereitgestellt sein, z.B. Tasten, ein berührungsempfindlicher Bildschirm, etc. Die Haarzustands-Ermittlungsvorrichtung kann in verschiedenen Ausführungsbeispielen eine Ausgabevorrichtung zum Ausgeben der Haarzustandsinformation und/oder der Empfehlung aufweisen, beispielsweise einen Lautsprecher und/oder ein Display, oder Leuchtindikatoren wie beispielsweise LEDs in verschiedenen Farben oder Anzahl, welche direkt sichtbar sind oder ein Symbol/Icon ausleuchten. In verschiedenen Ausführungsbeispielen kann die Haarzustands-Ermittlungsvorrichtung Teil eines Haarzustands-Ermittlungssystems sein, welches ferner zumindest eine Anzeigevorrichtung aufweisen kann, welche als Ausgabevorrichtung für die Haarzustandsinformation und/oder die Empfehlung genutzt werden kann.

Zum direkten oder indirekten Ermitteln des Haarzustands und ggf. zum direkten oder indirekten Ermitteln der Empfehlung (z.B. des Haarbehandlungsprodukts und/oder der Haarbehandlungsempfehlung) kann die Vorrichtung bzw. das System in verschiedenen Ausführungsbeispielen eine Datenverarbeitungsvorrichtung aufweisen.

In verschiedenen Ausführungsbeispielen, z.B. wenn die Anzeigevorrichtung ein Smartphone, Tablet o.ä. ist, kann die Anzeigevorrichtung ferner als Eingabevorrichtung und/oder als externe Datenverarbeitungsvorrichtung genutzt werden.

In verschiedenen Ausführungsbeispielen können bei dem Ermitteln der Empfehlung Daten und/oder Erfahrungswerte von (weiteren) Nutzern, welche einen ähnlichen Haarzustand (z.B. einen ähnlichen Schädigungsgrad und/oder eine ähnliche Haarfarbe) und ggf. ein ähnliches Profil (Alter, Geschlecht, Lebensgewohnheiten, Haartyp, etc.) aufweisen können, berücksichtigt werden. Ein breiter Daten- und/oder Erfahrungssatz kann dabei zu Hilfe genommen werden, um das Ergebnis zu optimieren. Das System kann ggf. als ein lernendes System gestaltet sein.

In verschiedenen Ausführungsbeispielen kann die Haarzustands-Ermittlungsvorrichtung eingerichtet sein, einen gegenwärtigen Haarschädigungszustand, welcher mittels des NIR-Sensors ermittelt worden ist, und eine gegenwärtige Haarfarbe eines Nutzers, beispielsweise mittels Messung sichtbaren Lichts oder UV-Lichts, welche mittels des Farbsensors ermittelt worden ist, von der Haarzustands-Ermittlungsvorrichtung einer Haarbehandlungsmittel-Mischvorrichtung zu übermitteln, welche eine individuelle Haarbehandlungsmittel-Zusammensetzung ermitteln und dem Nutzer ein entsprechendes Haarbehandlungsmittel, beispielsweise ein Shampoo oder eine Kur, bereitstellen kann.

In verschiedenen Ausführungsbeispielen kann mittels der Haarzustands-Ermittlungsvorrichtung eine standardisierte und objektive Beurteilung des Behandlungsergebnisses ermöglicht sein. Zu dem Zweck kann mittels des in der Vorrichtung enthaltenen optischen Sensors und ggf. weiteren Sensoren der Haarzustand eines Verbrauchers nach einem Befolgen der Empfehlung, z.B. nach einem Anwenden des empfohlenen Produkts und/oder nach einem Durchführen der empfohlenen Behandlung, ermittelt werden.

Dadurch ermöglichte kontinuierliche, ggf. häufige, Messungen verlässlicher Haarschädigungswerte können es dem Nutzer bzw. dem Verbraucher ermöglichen, den Gesundheitszustand und/oder Pflegezustand seiner Haare in einem zeitlichen Verlauf zu verfolgen und in einem Glauben an einen Erfolg der Behandlung bestärkt zu werden.

Die Haarzustands-Ermittlungsvorrichtung nach Anspruch 1 ist so eingerichtet , eine Haarschädigung als einen Gehalt an Cysteinsäure zu ermitteln. Der zugehörige optische Sensor ist geeignet, eine oder mehrere Aufnahmen in einem Nahinfrarotbereich (NIR-Bereich) durchzuführen.

In verschiedenen Ausführungsbeispielen kann ein weiterer optischer Sensor ein VIS-Sensor sein, der mit Hilfe von Fluoreszenz eine Haarschädigung bestimmen kann. Der Nahinfrarotbereich ist ein Wellenlängenbereich , in welchem geschädigtes Haar Absorptionsstrukturen aufweist, z.B. in welchem Cysteinsäure Licht absorbiert.

Ungeschädigtes Haar kann typischerweise einen Cysteinsäuregehalt in einem Bereich von etwa 0.5 % bis etwa 1 % (nach Gewicht) aufweisen. Bei Vorliegen einer Schädigung, beispielsweise infolge mehrfachen Ultrablondierens und/oder anderer Schädigungsmechanismen, kann der Cysteinsäuregehalt auf über 15 % (Gew.) ansteigen.

In der vorliegenden Erfindung wird diese Eigenschaft genutzt, um den Schädigungsgrad des Haars als einen Gehalt an Cysteinsäure zu quantifizieren.

In verschiedenen Ausführungsbeispielen kann geschädigtes Haar eine Eigenfluoreszenz zeigen, welche zusätzlich genutzt wird, um mittels Erfassens einer Fluoreszenzintensität des Haars den Schädigungsgrad zu ermitteln.

In verschiedenen Ausführungsbeispielen kann zum Ermitteln der Fluoreszenzintensität des Haars das Haar mittels der Lichtquelle mit UV-Licht (z.B. mit Licht in einem Wellenlängenbereich von etwa 315 nm bis etwa 380 nm) belichtet werden. Zu dem Zweck kann die Sensorvorrichtung mit einer UV-Lichtquelle versehen sein. Die UV-Lichtquelle kann eine UV-LED oder eine andere geeignete Lichtquelle sein, welche klein genug ist, um in der Vorrichtung und/oder in dem ersten Bereich der Vorrichtung untergebracht zu werden.

Während des Belichtens kann Fluoreszenzlicht registriert werden, welches von dem Haar emittiert wird. Die Fluoreszenzintensität kann anhand des registrierten Lichts ermittelt werden. Unter Einbeziehung der Fluoreszenzintensität des Haars kann dann der Schädigungsgrad des Haars ermittelt werden.

Dementsprechend kann ein optischer Sensor zumindest im Fluoreszenz-Wellenlängenbereich empfänglich sein. Der optische Sensor kann beispielsweise eine Kamera, ein Photometer, ein Colorimeter und/oder ein Spektrometer aufweisen oder sein. In verschiedenen Ausführungsbeispielen kann zwischen dem Haar und dem optischen Sensor in verschiedenen Ausführungsbeispielen ein Filter angeordnet sein.

Dank des technologischen Fortschritts der letzten Jahre können mittlerweile optische Sensoren (für den sichtbaren Wellenlängenbereich bzw. für den NIR- oder IR-Bereich) bereitgestellt werden, welche klein genug sind, um in einem Körper der Vorrichtung untergebracht zu werden.

In verschiedenen Ausführungsbeispielen, beispielsweise in einem Fall, dass für den optischen Sensor, z.B. einen Detektor des optischen Sensors und/oder optische Bauelemente wie ein dispergierendes Element eines Spektrometers o.ä., mehr Platz benötigt, als nahe dem Haar vorhanden ist, mindestens eine Lichtleitvorrichtung bereitgestellt sein. Dabei kann das Platz benötigende Bauelement (z.B. die Lichtquelle und/oder der Detektor) an einer Stelle der Haarzustands-Ermittlungsvorrichtung angeordnet sein, welche mehr Platz bereithält, und die mindestens eine Lichtleitvorrichtung kann so gestaltet sein, dass sie ein Leiten von Licht (z.B. des Lichts zum Beleuchten des Haars oder das Licht, welches mit dem Haar gewechselwirkt hat) zwischen dem Bauelement und einer Ein- bzw. Austrittsstelle des Lichts am Vorrichtungskörper leitet. Als Lichtleitvorrichtung können dabei herkömmliche für diesen Zweck bekannte Strukturen verwendet werden, z.B. Lichtleitfasern, Lichtleitkanäle, Spiegel und/oder sonstige optische Elemente, usw., wobei darauf zu achten ist, dass ein ggf. verwendetes lichtdurchlässiges Material für den jeweils zu leitenden Wellenlängenbereich lichtdurchlässig ist, z.B. im Fall des NIR-Lichts für den NIR-Wellenlängenbereich bis etwa 2,5 µm.

In verschiedenen Ausführungsbeispielen kann das Nahinfrarot- (NIR-) und/oder ein Infrarot- (IR-)Spektrum gewonnen werden, beispielsweise mittels ATR-(Nah-)Infrarotspektroskopie (von Englisch "attenuated total reflection", auf Deutsch "abgeschwächte Totalreflexion"). Durch eine Anwendung von mathematischen Modellen kann mittels Vermessung von Kalibrier-Haarproben, welche einen anhand eines bekannten analytischen Verfahrens ermittelten Cysteinsäuregehalt aufweisen, ein mathematisches Modell erstellt werden.

Bei einer Analyse eines am Haar eines Verbrauchers oder eines Nutzers aufgenommenen NIR- bzw. IR-Spektrums, bzw. zumindest eines Teils davon, kann in verschiedenen Ausführungsbeispielen das Modell eine Berechnung des Gehalts an Cysteinsäure, und damit der Haarschädigung, erlauben. Eine Analyse von zumindest einem Teil des Spektrums und eine Anwendung des Modells kann dabei mittels der Datenverarbeitungsvorrichtung, beispielsweise (mit geeigneten Apps) mittels bekannter Smartphones, Tablets o.ä., ausgeführt werden.

Die Lichtquelle kann in verschiedenen Ausführungsbeispielen eine NIR-Lichtquelle oder/und eine IR- Lichtquelle zum Belichten des Haars mit NIR- bzw. IR-Licht sein.

Das Ermitteln des Schädigungsgrads von Haar wird unter Nutzung des Nahinfrarot-Bereichs ausgeführt, zum Beispiel mittels Bestrahlens des Haars mit dem Nahinfrarotlicht und Spektralanalyse von zumindest einem Teil des NIR-Lichts, nachdem dieses mit dem Haar gewechselwirkt hat, oder unter Nutzung des Infrarot-Bereichs, d.h. mittels Bestrahlens des Haars mit Infrarotlicht und Spektralanalyse von zumindest einem Teil des IR-Lichts, nachdem dieses mit dem Haar gewechselwirkt hat, oder unter Verwendung von sowohl dem Nahinfrarot- als auch dem Infrarotbereich, d.h. mittels Bestrahlens des Haars mit Nahinfrarot- und Infrarotlicht und Spektralanalyse von zumindest einem Teil des NIR- und zumindest einem Teil des IR-Lichts, nachdem dieses mit dem Haar gewechselwirkt hat.

In verschiedenen Ausführungsbeispielen kann ein vermessener Nahinfrarot (NIR)-Bereich Wellenlängen von etwa 12500 cm-1 bis etwa 4000 cm-1, z.B. von etwa 5022 cm-1 bis etwa 4020 cm-1, aufweisen. Dieser Wellenlängenbereich kann u.a. charakteristische Oberton- und Kombinationsschwingungen von z.B. CH-, OH- und NH-Gruppen aufweisen.

In verschiedenen Ausführungsbeispielen kann der zumindest eine Teil des Nahinfrarot- und/oder Infrarotlichts einen (Infrarot-)Wellenzahlbereich von etwa 1100 cm-1 bis etwa 1000 cm-1, z.B. um etwa 1040 cm-1, aufweisen. Hier können sich u.a. die relevanten Absorptionsbanden der zu analysierenden Komponente Cysteinsäure befinden.

In verschiedenen Ausführungsbeispielen kann anhand von Ergebnissen einer quantitativen rechnergestützten Auswertung (auch als chemometrischen Analyse bezeichnet) für eine Mehrzahl von Kalibrationshaarproben in Kombination mit mittels eines unabhängigen Verfahrens, z.B. mittels Hochdruckflüssigchromatographie, für dieselben Kalibrationshaarproben gewonnenen Werten für einen Cysteinsäuregehalt der jeweiligen Kalibrationshaarprobe, ein Kalibrationsmodell erstellt werden.

Liegt das Kalibrationsmodell vor, kann in verschiedenen Ausführungsbeispielen sehr einfach für das zu messende Haar anhand des für das aufgenommene (N)IR-Spektrum die Konzentration der Cysteinsäure (als Maß für die Haarschädigung) aus den Spektren im Vergleich mit den Kalibrationsspektren berechnet werden.

In verschiedenen Ausführungsbeispielen können für die Quantifizierung des Cysteinsäuregehalts (z.B. mittels Fluoreszenzanalyse und/oder mittels (N)IR-Spektroskopie) bzw. des Schädigungsgrads geeignete mathematische Modelle der Prädiktiven Analytik genutzt werden.

In verschiedenen Ausführungsbeispielen wird ein in einer Nutzung einfaches Verfahren bereitgestellt, welches mit Hilfe von Fluoreszenz-Detektion und/oder durch Detektion von Absorption und Methoden aus der Prädiktiven Analytik eine präzise Ermittlung eines Grads einer oxidativen Schädigung von Haar ermöglicht.

In verschiedenen Ausführungsbeispielen kann für ein Bereitstellen des ermittelten Haarzustands bzw. der Empfehlung eine mobile bzw. tragbare Datenverarbeitungsvorrichtung verwendet werden. Als tragbare Datenverarbeitungsvorrichtung kann dabei beispielsweise ein Smartphone, ein iPad, ein Tablet oder Laptop genutzt werden.

In verschiedenen Ausführungsbeispielen kann ein Verfahren bereitgestellt werden, welches es ermöglicht, mittels einfacher bildanalytischer Verfahren, welche sich beispielsweise unter Verwendung einer einfachen Vorrichtung (z.B. einer UV-LED, einer Weißlicht-, NIR- und/oder IR-Leuchtvorrichtung, eines Filters, eines tragbaren NIR-Sensors, eines tragbaren (NIR- und/oder VIS-) Spektrometers, ggf. in Verbindung mit einer mobilen Datenverarbeitungsvorrichtung (z.B. eines Smartphones oder Tablets) und ggf. eines Prädiktive-Analytik-Verfahrens eine Information bezüglich eines Haarzustands eines Nutzers und ggf. eine darauf basierende Empfehlung bereitzustellen.

Gemäß verschiedenen Ausführungsbeispielen kann die prädiktive Analytik mindestens ein Verfahren nutzen aus einer Gruppe von Verfahren, wobei die Gruppe von Verfahren aufweist: lineare oder multi-lineare Regression, polynome Regression, neuronale-Netze-Verfahren, Support Vector Machine-Verfahren, Entscheidungsbäume-Verfahren ("Decision Trees", "Random Forest", "Tree Ensembles") und weitere Verfahren.

Mittels Verwendens eines Beschleunigungssensors kann es in verschiedenen Ausführungsbeispielen möglich sein, eine Position der Haarzustands-Ermittlungsvorrichtung zu ermitteln. Beispielsweise kann ein Beschleunigungssensor genutzt werden, um einen Beginn und/oder ein Ende eines Haarzustandserfassungsvorgangs zu ermitteln. Unter der Annahme, dass ein Haarzustandserfassungsvorgang typischerweise am Haaransatz beginnt und an den Haarspitzen endet, kann, ggf. in Kombination mit einer mittels des Beschleunigungssensors ermittelten Geschwindigkeit, mit welcher die Haarzustands-Ermittlungsvorrichtung bewegt wird, eine ortsaufgelöste Ermittlung, z.B. Haaransatz/mittlerer Bereich/Spitzen, der Haarzustandsinformation, Haarfarbe und dazugehörige Haarschädigung, ermöglicht sein.

Im Rahmen dieser Anmeldung kann das Merkmal Beschleunigungssensor einen "klassischen" Bewegungssensor oder einen gyroskopischen Sensor umfassen.

Eine Datenübermittlung von der Haarzustands-Ermittlungsvorrichtung zu einer externen Datenverarbeitungsvorrichtung und/oder zu einer externen Speichervorrichtung kann in verschiedenen Ausführungsbeispielen mittels Kabel oder über bekannte Datenfunkstandards (z.B. Bluetooth, Bluetooth Low Energy (BLE), WLAN, WiFi, NFC usw.) kabellos erfolgen.

Gemäß Anspruch 1 umfasst die Haarzustands-Ermittlungsvorrichtung zum Bereitstellen von Haarzustandsinformation *inter alia* einen ersten Bereich und einen zweiten Bereich, welche so eingerichtet sind, dass Haare eines Nutzers zwischen dem ersten Bereich und dem zweiten Bereich bewegbar sind, wobei in dem ersten Bereich mindestens ein optischer Sensor zum Erfassen von Licht einer Lichtquelle als die Haarzustandsinformation angeordnet ist, wobei der zweite Bereich mindestens einen Träger umfasst, und wobei der erste Bereich und der zweite Bereich mittels eines Verbindungselements derart gekoppelt sind, dass sich der erste Bereich und der zweite Bereich derart gegenüberliegen, dass eine Sensorhauptmessrichtung des mindestens einen Sensors in einem vordefinierten Winkel zu einer Oberfläche des Trägers ausgerichtet ist, aufweisen.

In verschiedenen Ausführungsbeispielen kann die Haarzustands-Ermittlungsvorrichtung mittels einer Hand von einem Benutzer bedient werden. Der Nutzer kann mit einer Hand mindestens ein Haar oder eine Haarsträhne freilegen und die Haare unter leichter für den Kunden noch angenehmer Zugbelastung der freigelegten Haare halten und mit der anderen Hand die Haarzustands-Ermittlungsvorrichtung entlang der freigelegten Haare führen.

In verschiedenen Ausführungsbeispielen kann der Nutzer mit einer Hand die Haarzustands-Ermittlungsvorrichtung nach einem vordefinierten Bewegungsmuster im Raum bewegen, um einhändig mindestens eine Aktion der Haarzustands-Ermittlungsvorrichtung zu steuern, beispielsweise einen Speichervorgang einer gerade gemessenen Haarfarbe und/oder eines gerade gemessenen Haarschädigungsgrads durchzuführen, beispielsweise einen Löschvorgang einer gerade erfassten und/oder in einer Speichervorrichtung gespeicherten Haarfarbe und/oder eines gerade erfassten und/oder in einer Speichervorrichtung gespeicherten Haarschädigungsgrads durchzuführen, beispielsweise einen Datenübermittlungsvorgang einer gerade erfassten und/oder in einer Speichervorrichtung gespeicherten Haarfarbe und/oder eines gerade erfassten und/oder in einer Speichervorrichtung gespeicherten Haarschädigungsgrads von der Haarzustands-Ermittlungsvorrichtung zu einer externen Datenverarbeitungsvorrichtung durchzuführen.

In verschiedenen Ausführungsbeispielen kann eine elektronische Schaltkreisvorrichtung eingerichtet sein, mittels eines Beschleunigungssensors zu ermitteln, ob eine Haarzustands-Ermittlungsvorrichtung über längeren Zeit hinweg nicht benutzt wurde und daraufhin die Haarzustands-Ermittlungsvorrichtung in einen Standby-Modus zu versetzen.

In verschiedenen Ausführungsbeispielen kann eine elektronische Schaltkreisvorrichtung eingerichtet sein, mittels eines Beschleunigungssensors zu ermitteln, ob eine Haarzustands-Ermittlungsvorrichtung über längeren Zeit hinweg nicht benutzt wurde und gerade benutzt wird und daraufhin die Haarzustands-Ermittlungsvorrichtung in einen Aktiv-Modus zu versetzen. In verschiedenen Ausführungsbeispielen kann beispielsweise abhängig von der Bewegungshäufigkeit der Haarzustands-Ermittlungsvorrichtung mittels einer Schaltkreisvorrichtung ein Ausschalten des Farbsensors und/oder des Haarschädigungssensors realisiert werden, um einen Leistungsverbrauch der Haarzustands-Ermittlungsvorrichtung in einem Standby-Modus herabzusetzen.

In verschiedenen Ausführungsbeispielen kann das Verbindungselement einen elastischen Werkstoff umfassen. In verschiedenen Ausführungsbeispielen zeichnet sich der elastische Werkstoff dadurch aus, dass er wiederholend bei Anlegen einer Kraft verformbar ist und bei Wegnehmen der Kraft in seinen ursprünglichen Ausgangszustand zurückkehrt.

In verschiedenen Ausführungsbeispielen kann das Verbindungselement eine Dicke bzw. Materialstärke in einem Bereich von etwa 2 mm bis etwa 20 mm umfassen.

In verschiedenen Ausführungsbeispielen kann das Verbindungselement ein gebogener Flachstahl mit einer Breite in einem Bereich von etwa 5 mm bis 30 mm umfassen.

In verschiedenen Ausführungsbeispielen kann das Verbindungselement einen Federstahl, beispielsweise X10CrNi18-8, 38Si7, 61SiCr7, 52CrMoV4, 51CrV4, C67E/C67S oder weitere dem Fachmann für derartige Anwendungen bekannte Federstähle, umfassen.

In verschiedenen Ausführungsbeispielen kann das Verbindungselement einen nichtrostenden Federstahl umfassen.

In verschiedenen Ausführungsbeispielen kann das elastische Verbindungsmaterial mattiert und/oder matt lackiert ausgestaltet sein, was es ermöglichen kann, dass ein von einer Lichtquelle abgesendetes Licht nur gering bzw. in einem vordefinierten Bereich reflektiert werden kann und eine negative Beeinflussung mindestens eines Sensors minimiert werden kann.

In verschiedenen Ausführungsbeispielen kann das Verbindungselement eine U-förmige Ausgestaltung aufweisen und der erste Bereich und der zweite Bereich jeweils an einem Endbereich der U-förmigen Ausgestaltung positioniert sein.

In verschiedenen Ausführungsbeispielen kann das Verbindungselement nahe des und/oder neben dem zweiten Bereich(s) eine Anschlagsbegrenzung umfassen, wobei die Anschlagsbegrenzung eingerichtet ist, dass bei einem Zusammendrücken des Verbindungselements mittels Handmuskelkraft eine zu hohe Kraftbelastung durch den Träger und/oder das Kalibriermaterial auf eine Sensorfrontseite vermieden werden kann.

In verschiedenen Ausführungsbeispielen kann auf einem dem ersten Bereich zugewandten Bereich der Oberfläche des Trägers mindestens ein Kalibriermittel angeordnet sein, wobei das Kalibriermittel eingerichtet ist, zumindest einen Sensor der Vorrichtung zu kalibrieren.

In verschiedenen Ausführungsbeispielen kann der Aufbau der Vorrichtung es ermöglichen, dass das Kalibriermittel Teil der Vorrichtung ist und somit keine zusätzlichen Kalibriermittel für einen Kalibriervorgang mindestens eines benötigt wird.

In verschiedenen Ausführungsbeispielen kann es ermöglicht sein, dass das Kalibriermittel bereits an der Vorrichtung angebracht ist und verhindert werden kann, dass bei einem mobilen Einsatz der Vorrichtung ein Kalibriermittel nicht zur Hand liegt, so dass eine Kalibrierung nicht möglich wäre. In verschiedenen Ausführungsbeispielen kann es ermöglicht werden, dass an jedem beliebigen Ort, an dem die Vorrichtung kalibriert werden soll, ein Kalibriermittel, das mit der Vorrichtung physikalisch verbunden ist, zur Verfügung steht und kein externes Kalibriermittel benötigt wird.

In verschiedenen Ausführungsbeispielen kann das Kalibriermittel bzw. eine Oberfläche des Kalibriermittels bei einer Durchführung einer Kalibrierung senkrecht oder in einem vordefinierten Winkel zu einer Hauptmessrichtung des mindestens einen Sensors positioniert sein. In verschiedenen Ausführungsbeispielen kann der vordefinierte Winkel in einem Bereich von etwa 70 Grad bis etwa 110 Grad, beispielsweise in einem Bereich von etwa 85 Grad bis etwa 95 Grad liegen.

In verschiedenen Ausführungsbeispielen kann ein seitlicher Abstand einer senkrechten Mittelachse des Kalibriermittels zu einer senkrechten Mittelachse bzw. Längsachse mindestens eines Sensors in einem vordefinierten Bereich, beispielsweise in einem Bereich von etwa 20 mm bis etwa 0 mm, beispielsweise in einem Bereich von etwa 10 mm bis 0 mm, liegen.

In verschiedenen Ausführungsbeispielen kann das Kalibriermittel als ein Referenzkalibriermittel verwendet werden, um den Farbsensor und/oder den Haarzustandssensor zu kalibrieren.

In verschiedenen Ausführungsbeispielen kann ein Material des Trägers und/oder der Keramik in der Farbe Weiß bzw. in einem Weiß-Farbton gehalten werden, um eine hohe Reflexion von Licht zu ermöglichen. In verschiedenen Ausführungsbeispielen kann das Keramikmaterial bzw. der Keramikwerkstoff eine Glaskeramik und/oder leuzitverstärkte Glaskeramik umfassen. In verschiedenen Ausführungsbeispielen kann eine leuzitverstärkte Glaskeramik eine Glasphase und eine Kristallphase vom Typ des Leuzits umfassen. In verschiedenen Ausführungsbeispielen kann das Material beispielweise auf dem Dreistoffsystem SiO¹, Al²O³ und K²O basieren.

In verschiedenen Ausführungsbeispielen kann als das Keramikmaterial beispielsweise ALOTEC 92 verwendet. In verschiedenen Ausführungsbeispielen kann ALOTEC 92 mit einem Al₂O₃-Gehalt von größer oder gleich 92 Masse-%, einer Dichte von größer 3,64 g/cm³, einer offenen Porosität von etwa 0 Vol-%, einem E-Modul von größer 300, einer Biegesteifigkeit (Vier-Punkt) von größer 320 MPa, einem Weibull-Modul von etwa 14 m, einer Bruchzähigkeit von etwa 3 bis 4 MPam^{V2}, einer Härte von größer 12 HV(1), einem Wärmeausdehnungskoeffizienten von 7,5 10⁻¹K⁻¹, einer Rauigkeit "as fired" von etwa 1,9 µm und einer Rauigkeit "poliert" von etwa 0,3 µm verwendet werden. Es versteht sich jedoch von selbst, dass anstelle von ALOTEC 92 weitere geeignete Keramikmaterialen verwendet werden können.

In verschiedenen Ausführungsbeispielen kann bei einer Herstellung einer Glaskeramik ausgehend von einem viskosen Glas zunächst die Form hergestellt und in einem anschließenden Temperschritt, d.h. einem Verfahren zur Stabilitätssteigerung der Keramik, durch den gezielten Einsatz von Temperaturänderungen, das vorher amorphe Volumen gezielt auskristallisiert werden. Solche Systeme zeichnen sich durch eine hohe mechanische Festigkeit sowie durch eine gute Temperaturwechselbeständigkeit aus. In verschiedenen Ausführungsbeispielen kann es ermöglicht werden, durch den Einsatz einer leuzitverstärkten Glaskeramik als Kalibriermittel die positiven optischen Eigenschaften von Gläsern mit den positiven mechanischen Eigenschaften von Keramiken zu kombinieren.

In verschiedenen Ausführungsbeispielen kann der NIR-Sensor bei einem Kalibriervorgang mittels des Kalibriermittels nach einem Aktivieren des NIR-Sensors in einen Kalibriermodus gebracht werden. In verschiedenen Ausführungsbeispielen kann dann das Kalibriermittel in einem vordefinierten Abstand zu dem NIR-Sensor an den NIR-Sensor gebracht werden. In verschiedenen Ausführungsbeispielen kann bei einem Kalibriervorgang des NIR-Sensors das Kalibriermittel direkt vor oder direkt an eine Hauptmessoberfläche des Farbsensors und/oder des NIR-Sensors positioniert werden. In verschiedenen Ausführungsbeispielen kann dann der NIR-Sensor mittels einer Infrarotquelle (IR-Quelle) das Kalibriermittel, beispielsweise eine Keramik, bestrahlen und eine Reflektion (und dadurch auch eine Absorption) der reflektierten Infrarotstrahlen messen. In verschiedenen Ausführungsbeispielen kann der NIR-Sensor dann ein internes Kalibrierprofil für alle weiteren Messungen generieren.

In verschiedenen Ausführungsbeispielen kann der Farbsensor einen selbstkalibrierenden Sensor umfassen. In verschiedenen Ausführungsbeispielen kann der Farbsensor optional mittels des Kalibriermittels kalibriert werden.

In verschiedenen Ausführungsbeispielen kann der Farbsensor bei einem Kalibriervorgang mittels des Kalibriermittels nach einem Aktivieren des Farbsensors in einen Kalibriermodus gebracht werden. In verschiedenen Ausführungsbeispielen kann dann das Kalibriermittel in einem vordefinierten Abstand zu dem Farbsensor an den Farbsensor, beispielsweise mittels Muskelkraft des Nutzers, positioniert werden. In verschiedenen Ausführungsbeispielen kann bei einem Kalibriervorgang des Farbsensors das Kalibriermittel direkt vor oder direkt an eine Hauptmessoberfläche des Farbsensors positioniert werden. In verschiedenen Ausführungsbeispielen kann dann der Farbsensor mittels einer Infrarotquelle (IR-Quelle) das Kalibriermittel, beispielswiese eine Keramik, bestrahlen und eine Reflektion der reflektierten Lichtstrahlen messen. In verschiedenen Ausführungsbeispielen kann der Farbsensor dann ein internes Kalibrierprofil für alle weiteren Messungen generieren. In verschiedenen Ausführungsbeispielen kann eine Kalibrierung eines selbstkalibrierenden Farbsensors optional zum besseren Einmessen der Lichtquelle für besonders präzise Messungen notwendig sein.

In verschiedenen Ausführungsbeispielen kann ein Kalibriervorgang mittels des Kalibriermittels als Teil der Vorrichtung vereinfacht werden.

In verschiedenen Ausführungsbeispielen kann das Kalibriermittel oder der Träger ferner als Klemmvorrichtung und/oder Führungshilfe eingerichtet sein, so dass Haar eines Verbrauchers in Richtung der Messsensoren geführt werden kann. Anders ausgedrückt, kann in verschiedenen Ausführungsbeispielen der Träger und/oder das Kalibriermittel mittels Handmuskelkraft eines Nutzers hin zu dem mindestens einen Sensor geführt werden, um bei dem mindestens einen Sensor eine Messung bei einem vordefinierten Abstand, beispielsweise in einem Bereich von etwa 0 mm bis etwa 10 mm, beispielweise in einem Bereich von etwa 2 mm bis etwa 8 mm, beispielsweise in einem Bereich von etwa 3 mm, zwischen dem Träger oder dem Kalibriermittel und dem mindestens einen Sensor durchführen zu können.

In verschiedenen Ausführungsbeispielen kann das Kalibriermittel ein Keramikmaterial umfassen oder sein.

In verschiedenen Ausführungsbeispielen kann es ermöglicht werden, dass mittels der Keramik ein Hintergrund hinter den zu beleuchtenden Haaren eines Verbrauchers konstant gehalten werden kann, so dass wiederholbare bzw. reproduzierbare Messungen mit der Haarzustands-Ermittlungsvorrichtung erreich werden können.

In verschiedenen Ausführungsbeispielen kann das Kalibriermittel eine Keramikplatte sein, die auf einem Träger aufgebracht ist.

In verschiedenen Ausführungsbeispielen kann der erste Bereich einen Farbsensor zum Erfassen einer Haarfarbe eines Nutzers und/oder einer Haarschädigung über UV-Licht mittels einer Absorption oder Reflektion und einen Haarzustandssensor zum Erfassen eines Haarzustands des Nutzers umfassen, wobei der Farbsensor zu einer ersten Zeit eine Haarfarbe des Nutzers erfasst und der Haarzustandssensor zu einer zweiten Zeit einen Haarschädigungsgrad und/oder Haarfeuchtigkeitsgehalt des Nutzers erfasst, und wobei die erste Zeit und die zweite Zeit in einem vordefinierten Zeitbereich liegen.

In verschiedenen Ausführungsbeispielen kann der erste Bereich ferner einen haptischen Signalgeber (Vibrator) umfassen.

In verschiedenen Ausführungsbeispielen kann der Farbsensor eingerichtet sein, eine aktuelle Haarfarbe eines Nutzers zu erfassen.

In verschiedenen Ausführungsbeispielen kann der Farbsensor ein High-End-Multi-Band-Sensor bzw. Farbsensor mit hoher Bandbreite oder ein Multi-Spektral-Farbsensor umfassen oder sein.

In verschiedenen Ausführungsbeispielen kann ein Farbsensor mit hoher Bandbreite und/oder erweitertem Wellenspektrum für die Erfassung dafür eingerichtet sein, zusätzliche Farbinformationen neben dem vom menschlichen Auge wahrgenommenen Farbspektrum für die weitere Analyse und Berechnung von Pflege und Färbungsprodukten zur Verfügung zu stellen.

In verschiedenen Ausführungsbeispielen kann mittels des Farbsensors eine Mehrkanal-Farbmessung mit Transformation in einen Darstellungsraum, beispielsweise einen CIE LAB oder in weitere dem Fachmann bekannte Darstellungsräume, realisiert sein.

In verschiedenen Ausführungsbeispielen kann hierin ein L*a*b*-Farbraum verstanden werden als Farbraum, bei dem alle Farben in geräteunabhängiger Form enthalten sind. Mittels einer Übertragung von Farbwerten in den L*a*b*-Farbraum kann daher die verlustfreie Konvertierung von Farbinformationen aus einem Farbsystem in ein anderes, von einer Geräteart in eine andere, realisiert sein.

In verschiedenen Ausführungsbeispielen kann der vordefinierte Zeitbereich beispielsweise in einem Bereich von etwa 10 ms (Millisekunden) bis etwa 1000 ms liegen. In verschiedenen Ausführungsbeispielen kann die erste Zeit beispielsweise in einem Bereich von etwa 20 ms bis 50 ms und die zweite Zeit in einem Bereich von etwa 50 ms bis 250 ms liegen und umgekehrt. Anders ausgedrückt, kann beispielsweise mittels des Farbsensors eine Messung durchgeführt werden und kurz darauf mittels des Haarzustandssensors eine Messung durchgeführt werden. In verschiedenen Ausführungsbeispielen kann mittels des Haarzustandssensors eine Messung durchgeführt werden und kurz darauf mittels des Farbsensors eine Messung durchgeführt werden. In verschiedenen Ausführungsbeispielen können die Messungen so dicht nacheinander durchgeführt werden, dass sie annähernd zur gleichen Zeit durchgeführt werden können.

In verschiedenen Ausführungsbeispielen kann ein erstes erfasstes Messergebnis mit dem Farbsensor und ein zweites Messergebnis mit dem Haarzustandssensor und/oder ein drittes Messergebnis mit dem Beschleunigungssensor zumindest gemeinsam in einem Takt, beispielsweise einem Speichertakt der elektronischen Schaltkreisvorrichtung, verarbeitet werden und/oder zu einem Datenpaket zusammengeführt werden, welches beispielsweise mittels der Schaltkreisvorrichtung in der Speichervorrichtung der Haarzustands-Ermittlungsvorrichtung und/oder in einer externen Speichervorrichtung und/oder in einer externen Datenverarbeitungsvorrichtung mittels einer Drahtlosverbindung gespeichert werden kann.

In verschiedenen Ausführungsbeispielen kann der Farbsensor eingerichtet sein, eine aktuelle Haarfarbe eines Verbrauchers zu erfassen.

In verschiedenen Ausführungsbeispielen kann der erste Bereich oder der zweite Bereich ferner einen Beschleunigungssensor umfassen.

In verschiedenen Ausführungsbeispielen kann mit dem Begriff Beschleunigungssensor ein Beschleunigungsmesser, Accelerometer, Akzelerometer, B-Messer oder G-Sensor gemeint sein.

In verschiedenen Ausführungsbeispielen kann die Beschleunigung in der SI-Einheit m·s⁻² (Meter pro Sekunde zum Quadrat) gemessen bzw. angegeben.

In verschiedenen Ausführungsbeispielen kann der Beschleunigungssensor eingerichtet sein, eine Bewegung und/oder eine Geschwindigkeit einer Vorwärtsbewegung der Haarzustands-Ermittlungsvorrichtung durch einen Nutzer zu erfassen.

In verschiedenen Ausführungsbeispielen kann der Beschleunigungssensor eingerichtet sein, eine Bewegung und/oder eine Geschwindigkeit einer Vorwärtsbewegung der Haarzustands-Ermittlungsvorrichtung durch einen Nutzer zu erfassen, beispielsweise wenn unterschiedliche Haarbereiche des Haars mittels der Haarzustands-Ermittlungsvorrichtung vermessen werden, um eine momentane Position der Haarzustands-Ermittlungsvorrichtung in Bezug auf das Haar, beispielsweise in Bezug auf ein Haarbeginn oder ein Haarende, zu erfassen und/oder, um eine Bewegungsrichtung und/oder eine Bewegungsgeschwindigkeit der Haarzustands-Ermittlungsvorrichtung zu erfassen.

In verschiedenen Ausführungsbeispielen kann der Beschleunigungssensor eingerichtet sein, einen Bewegungszustand der Vorrichtung im Raum zu erfassen. In verschiedenen Ausführungsbeispielen kann mittels des Verwendens eines Beschleunigungssensors zusätzlich zu weiteren Sensoren, beispielsweise zu einem Farbsensor und einem Haarzustandssensor, ermöglicht werden, dass die Haarzustands-Ermittlungsvorrichtung einhändig bedient werden kann, indem ein Betrieb der Haarzustands-Ermittlungsvorrichtung mittels einer physikalischen Bewegungs- und Gestensteuerung gesteuert werden kann.

In verschiedenen Ausführungsbeispielen kann der Beschleunigungssensor eingerichtet sein, eine vordefinierte "Hin-und-her"-Bewegung und/oder Schwenkbewegung und/oder "Schüttel"-Bewegung und/oder "Ausschüttel"-Bewegung und/oder "Zeigebewegung" der Haarzustands-Ermittlungsvorrichtung zu erfassen und/oder mit einem vordefinierten Bewegungsmuster zu vergleichen und/oder eine Bewegung einem bekannten Bewegungsmuster zuzuordnen.

In verschiedenen Ausführungsbeispielen kann nach einem Greifen der Vorrichtung und einem Herausnehmen der Vorrichtung aus einer Ladestation und anschließendem Senkrechthalten der Vorrichtung der Beschleunigungssensor eingerichtet sein, die senkrechte Ausrichtung der Vorrichtung zu erfassen, wobei eine Schaltkreisvorrichtung eingerichtet ist, basierend auf mindestens einem Messergebnis des Beschleunigungssensors ein Vergleich des Messergebnisses mit vordefinierten bekannten Bewegungsmustern durchzuführen und ein der Bewegung entsprechendes vordefiniertes Bewegungsmuster zu ermitteln und ein Einschalten bzw. Aufwecken der Vorrichtung durchzuführen.

In verschiedenen Ausführungsbeispielen kann bei einem Senkrechthalten der Vorrichtung und Schütteln der Vorrichtung der Beschleunigungssensor eingerichtet sein, die senkrechte Ausrichtung der Vorrichtung und das Schütteln zu erfassen, wobei eine Schaltkreisvorrichtung eingerichtet sein kann, basierend auf mindestens einem Messergebnis des Beschleunigungssensors ein Vergleich des Messergebnisses mit vordefinierten bekannten Bewegungsmustern durchzuführen und ein der Bewegung entsprechendes vordefiniertes Bewegungsmuster zu ermitteln und basierend auf dem ermittelten vordefinierten Bewegungsmuster eine neue Messserie zu aktivieren bzw. zu beginnen.

In verschiedenen Ausführungsbeispielen kann bei einem Ausschütteln der Vorrichtung der Beschleunigungssensor eingerichtet sein, das Schütteln als Bewegungsmuster zu erfassen, wobei eine Schaltkreisvorrichtung eingerichtet ist, basierend auf dem erfassten Bewegungsmuster mittels des Beschleunigungssensors ein Vergleich des Bewegungsmusters mit vordefinierten bekannten Bewegungsmustern durchzuführen und ein der Bewegung entsprechendes vordefiniertes Bewegungsmuster zu ermitteln und basierend auf dem ermittelten vordefinierten Bewegungsmuster eine letzte Messung bzw. eine letzte Messreihe aus einer Speichervorrichtung, beispielsweise aus einer Speichervorrichtung der Haarzustands-Ermittlungsvorrichtung und/oder aus einer Speichervorrichtung einer externen Datenverarbeitungsvorrichtung und/oder aus einer externen Datenbank, zu löschen.

In verschiedenen Ausführungsbeispielen kann die Vorrichtung eingerichtet sein, ein gleichzeitiges Messen mittels eines NIR-Sensors und eines Farbsensors und/oder eines Bewegungssensors bzw. ein gleichzeitiges Speichern von entsprechenden Messwerte durchzuführen, damit zu einem erfassten Haarschädigungsgrad eine jeweilige erfasste Farbe von Haar und/oder eine Position am Haar eindeutig zugeordnet und in einer Speichervorrichtung archiviert werden kann. In verschiedenen Ausführungsbeispielen kann eine Position am Haar einen oberen Bereich am Haaransatz, einen mittleren Bereich und einen unteren Bereich, oder einen Randbereich umfassen. In verschiedenen Ausführungsbeispielen kann beispielsweise ein oberer Bereich von Haar eines Verbrauchers am Haaransatz einen Braunton bei einem niedrigen bis geringen Haarschädigungsgrad des Haars und ein unterer Randbereich des Haars eines Nutzers an den Spitzen einen hellen Blondton mit einer mittleren bis starken Haarschädigungsgrad aufweisen.

In verschiedenen Ausführungsbeispielen kann durch das gleichzeitige Messen mittels eines NIR-Sensors und eines Farbsensors und/oder durch das Erfassen einer Bewegung der Vorrichtung mittels des Bewegungssensors ermöglicht werden, dass eine schnelle Bedienung der Vorrichtung durch konsekutive Messdurchgänge realisierbar ist.

In verschiedenen Ausführungsbeispielen kann durch das gleichzeitige Messen mittels eines NIR-Sensors und eines Farbsensors und/oder durch das Erfassen einer Bewegung der Vorrichtung mittels des Bewegungssensors ermöglicht werden, dass eine eindeutige Zuordnung der Infrarotmessung mittels des NIR-Sensors und einer Messung des sichtbaren Lichts mittels des Farbsensors zur einer Position an Haar eines Verbrauchers realisierbar ist.

In verschiedenen Ausführungsbeispielen kann durch das gleichzeitige Messen mittels eines NIR-Sensors und eines Farbsensors und/oder durch das Erfassen einer Bewegung der Vorrichtung mittels des Bewegungssensors ermöglicht werden, dass lokalisierte Messzonen mit gleichzeitiger Infrarotwert- und Farbwertbestimmung realisierbar sind, um anhand mehrerer Messungen des Haars die historische Beschädigung bzw. Veränderung des Haars zu dokumentieren.

In verschiedenen Ausführungsbeispielen kann als ein Beschleunigungssensor beispielsweise ein miniaturisierter, aus Silizium gefertigter piezoelektrischer Beschleunigungssensor verwendet wird, welcher die von einer Beschleunigung, d.h. vorlegend durch die Beschleunigung der Haarzustands-Ermittlungsvorrichtung mit einer Handbewegung, verursachten Druckschwankungen in elektrische Signale umwandeln kann.

In verschiedenen Ausführungsbeispielen kann als ein Beschleunigungssensor beispielsweise ein piezoresistiver oder piezokapazitiver oder mikromechanischer gyroskopischer Sensor verwendet werden, der ein Signal liefert, das neben der Beschleunigung auch die Inklination des Sensors (Lage in Bezug zur Gravitation) zeigen kann.

In verschiedenen Ausführungsbeispielen kann beispielsweise ein piezokeramisches Sensorplättchen dynamische Druckschwankungen in elektrische Signale umwandeln, die entsprechend weiterverarbeitet werden können. Die Druckschwankungen werden durch eine an der Piezokeramik befestigte (seismische) Masse erzeugt und können bei einer Beschleunigung des Gesamtsystems auf die Piezokeramik wirken.

In verschiedenen Ausführungsbeispielen können mikro-elektro-mechanische Systeme (MEMS) als Beschleunigungssensor eingesetzt werden, welche aus Silizium hergestellt sein können. In verschiedenen Ausführungsbeispielen können die Sensoren Feder-Masse-Systeme darstellen, bei denen die Federn wenige µm breite Silizium-Stege sein können und auch die Masse aus Silizium hergestellt werden kann. Durch die Auslenkung bei Beschleunigung kann zwischen dem gefedert aufgehängten Teil und einer festen Bezugselektrode eine Änderung der elektrischen Kapazität gemessen werden. Der gesamte Messbereich kann einer Kapazitätsänderung von ca. 1 pF entsprechen. Die Elektronik zur Auswertung dieser kleinen Kapazitätsänderung kann auf einem integrierten Schaltkreis (IC) untergebracht sein.

In verschiedenen Ausführungsbeispielen kann der Beschleunigungssensor derart eingerichtet sein, dass sich bei horizontaler oder vertikaler Lage Gleichspannungsanteile des Sensormesssignals unterscheiden, so dass auch die Position des Körpers, beispielsweise der Haarzustands-Ermittlungsvorrichtung, im Raum bestimmt werden kann.

In verschiedenen Ausführungsbeispielen kann es, da ein Beschleunigungssensor nur in einer Dimension mit maximaler Empfindlichkeit reagieren kann, nötig sein, dass eine Mehrzahl von Sensoren, beispielsweise zwei oder drei Sensoren kombiniert werden müssen, um Bewegungen in der Ebene oder im dreidimensionalen Raum erfassen zu können. In verschiedenen Ausführungsbeispielen können eine Mehrzahl von Sensoren verwendet werden, um ein menschliches Bewegungsverhalten in den drei räumlichen Dimensionen (Ebenen) genauer messen zu können.

In verschiedenen Ausführungsbeispielen kann der Beschleunigungssensor eingerichtet sein, Translationsbewegung und Rotationsbewegungen und eine Mischung von Beidem der Vorrichtung bei einer vordefinierten Abtastrate zu erfassen.

In verschiedenen Ausführungsbeispielen kann ein Aktivitäts- bzw. Mustererkennungsverfahren aus mehreren aufeinanderfolgenden Verarbeitungsschritten aufgebaut sein. In verschiedenen Ausführungsbeispielen kann eine Verarbeitung von mittels des Beschleunigungssensors erfassten Messdaten die Aufnahme der Rohsensordaten, deren Vorverarbeitung und Segmentierung, das Gewinnen aussagekräftiger Merkmale und das eigentliche Klassifizieren, das die aufgenommenen Daten den einzelnen Aktivitäten zuordnen kann, umfassen. In verschiedenen Ausführungsbeispielen kann es ein Ziel sein, aus einer Menge von Aktivitäten bzw. Bewegungsabläufen denjenigen Bewegungsablauf zu ermitteln, welcher die gemessenen Sensordaten am besten erklären kann. In verschiedenen Ausführungsbeispielen kann für eine Bewegungserfassung ein Messbereich von etwa ±5 G, beispielsweise von etwa ±4 G, und eine Abtastrate in einem Bereich von etwa 20 bis 60 Hz, beispielsweise von etwa 50 Hz, ausgewählt werden.

In verschiedenen Ausführungsbeispielen kann der Beschleunigungssensor bereits digital und zu einem gewissen Grad "intelligent" sein, so dass die meisten Vorverarbeitungsschritte direkt auf der Hardware selbst durchgeführt werden können, beispielsweise eine Analog-Digital-Wandlung, eine Kalibrierung oder eine Temperaturkompensation. In verschiedenen Ausführungsbeispielen kann es sinnvoll sein, die gemessenen Sensorrohdaten in standardisierte Einheiten, z. B. m/s² oder rad/s, und in Koordinatensysteme umzurechnen. In verschiedenen Ausführungsbeispielen können, wenn mehrere Sensoren verwendet werden, die Datenströme darüber hinaus synchronisiert werden. In verschiedenen Ausführungsbeispielen kann das nicht nur einen statischen Versatz zwischen den Signalen betreffen, sondern kann für verschiedene Abtastraten ein komplettes Resampling erforderlich machen.

In verschiedenen Ausführungsbeispielen kann der mindestens eine Beschleunigungssensor einen kontinuierlichen Datenstrom liefern. Um herausfinden zu können, in welchem Zeitraum welche Bewegung bzw. Aktivität stattgefunden hat, können in verschiedenen Ausführungsformen aus dem Strom einzelne Segmente extrahiert werden. So kann in verschiedenen Ausführungsbeispielen versucht werden, bereits einen Anfang und ein Ende möglicher Aktivitäten - etwa durch Bestimmung der Intensität der Bewegung - zu detektieren oder es können zusätzliche Sensoren oder Kontextinformationen zu Hilfe genommen werden. In verschiedenen Ausführungsbeispielen kann ein Fenster fester Größe in bestimmter Schrittweite und/oder überlappend über die Daten geschoben werden (Sliding Window). In verschiedenen Ausführungsbeispielen kann darauf zu achten sein, dass die Segmentgröße nicht zu klein, aber auch nicht zu groß gewählt wird. Wenn sie zu klein ist, könnten prägnante Bewegungsmuster abgeschnitten werden. Ist sie zu groß, könnten bereits Muster mehrerer aufeinanderfolgender Bewegungen verschwimmen. In verschiedenen Ausführungsbeispielen können die Messungen zusätzlich je nach Position im Segment gewichtet werden, beispielsweise als Hamming Window. In verschiedenen Ausführungsbeispielen kann für ein Sliding Window der Größe von 128 Samples, bei beispielsweise 50 Hz ca. 2,56 s mit Rechteckfenstern (ungewichtet) verwendet werden, die halb überlappend übereinander geschoben werden können. In verschiedenen Ausführungsbeispielen kann beispielsweise ein Ringspeicher eingesetzt werden, der zunächst gefüllt werden kann und dann alle Samples ausgelesen und weiterverarbeitet werden können.

In verschiedenen Ausführungsbeispielen kann bei der Merkmalsberechnung und der Selektion derart vorgegangen werden, dass aus dem von den Sensoren gemessenen Ausgangssignalen aussagekräftige Merkmale berechnen werden können, anhand derer die gesuchten Aktivitäten möglichst gut voneinander unterschieden werden können. Merkmale können dann für jedes Segment berechnet werden und als Merkmalsvektor an ein Klassifizierungsverfahren weitergereicht werden. Der von den Merkmalsvektoren aufgespannte Raum kann Merkmalsraum genannt werden, je mehr Merkmale pro Segment berechnet werden, desto größer oder höherdimensionaler kann der Merkmalsraum sein. Für Merkmalsselektion und -reduktion können in verschiedenen Ausführungsbeispielen automatische Heuristiken und Methoden zu Verfügung stehen wie etwa die Hauptkomponentenanalyse. Etablierte Merkmale können beispielsweise statistische Momente wie Mittelwert, Varianz, Schiefe und Wölbung umfassen. Frequenzbasierte Merkmale können mittels FFT, wie Peak oder Energie in Frequenzbändern, Körpermodellparameter wie Gelenkwinkel und Orientierungen oder noch abstraktere Varianten realisiert sein.

In verschiedenen Ausführungsbeispielen kann die Erkennung menschlicher Bewegungen mittels einfachen ad hoc zusammengestellten Regelsystemen über String Matching und Dynamic-Time-Warping-Verfahren (DTW) aus der Spracherkennung, Conditional Random Fields (CRF), zeitbehafteten probabilistischen Modellen wie Hidden Markov Models (HMM), dynamischen Bayesschen Netzen (DBN) oder Computational State Space Models (CSSM) realisiert sein. In verschiedenen Ausführungsbeispielen können für eine Erkennung einfacher Bewegungen diskriminative statistische Maschinenlernverfahren, beispielsweise Support Vector Machines (SVM) und C4.5-Entscheidungsbäume, verwendet werden.

In verschiedenen Ausführungsbeispielen kann die Vorrichtung ferner eine elektronische Schaltkreisvorrichtung, beispielsweise einen Prozessor, umfassen.

In verschiedenen Ausführungsbeispielen kann die Schaltkreisvorrichtung zumindest eingerichtet sein, basierend auf zumindest einem mittels des Beschleunigungssensors erfassten Bewegungszustand der Vorrichtung einen Betriebszustand der Vorrichtung zu steuern.

In verschiedenen Ausführungsbeispielen kann die Schaltkreisvorrichtung basierend auf dem jeweiligen erfassten Bewegungszustand ferner eingerichtet sein, zumindest eine mittels des mindestens einen Sensors erfasste Haarzustandsinformation in einer Speichervorrichtung der Vorrichtung oder in einer externen Speichervorrichtung zu speichern, und/oder mindestens eine Haarzustandsinformation aus der Speichervorrichtung der Vorrichtung oder aus der externen Speichervorrichtung zu löschen, und/oder die Haarzustandsinformation an eine externe Datenverarbeitungsvorrichtung zu übermitteln, und/oder Information basierend auf der Haarzustandsinformation auf einer Anzeigevorrichtung der Vorrichtung und/oder auf einer Anzeigevorrichtung der externen Datenverarbeitungsvorrichtung anzuzeigen.

In verschiedenen Ausführungsbeispielen kann die Vorrichtung ferner eine Anzeigevorrichtung zum Anzeigen von Information basierend auf der mittels der Vorrichtung erfassten mindestens einen Haarzustandsinformation umfassen.

In verschiedenen Ausführungsbeispielen kann die Vorrichtung ferner eine Vibrationseinheit beinhalten, um den Abschluss einer Messung, Fehlerzustände oder weitergehende Systemzustände haptisch an den Anwender mitzuteilen.

In verschiedenen Ausführungsbeispielen kann eine Eingabevorrichtung genutzt werden, um ein Wunschergebnis einzugeben, beispielsweise eine Wunschhaarfarbe, einen Wunsch-Pflegezustand, oder ein gewünschtes Styling. Darüber hinaus kann die Eingabevorrichtung genutzt werden, um der Haarzustands-Ermittlungsvorrichtung weitere Informationen bereitzustellen, welche ggf. beim Ermitteln der Empfehlung berücksichtigt werden können, z.B. ob ein Produkt ggf. wasserfest sein soll, Alter und/oder Geschlecht des Nutzers (z.B. für einen Fall, dass ein Pflegeprodukt mit einem Duft versehen ist), usw.

In verschiedenen Ausführungsbeispielen können die Sensoren gleichzeitig betrieben werden.

In verschiedenen Ausführungsbeispielen kann der Haarzustandssensor neben den in Anspruch 1 definierten Sensoren weitere Sensoren umfassen, z.B. einen Infrarotsensor (IR-Sensor) und/oder einen UV/Fluoreszenzsensor und/oder einen weiteren Sensor, mittels dessen sichtbares Licht erfassbar ist.

In verschiedenen Ausführungsbeispielen kann mittels des Haarzustandssensors eine innere Haarschädigung und/oder ein Feuchtigkeitslevel des Haars erfasst und mittels einer elektronischen Schaltkreisvorrichtung ermittelt werden.

In verschiedenen Ausführungsbeispielen kann die Haarzustands-Ermittlungsvorrichtung Teil eines Ecosystems aus Geräten und/oder Softwareprodukten sein. Ein solches Ecosystem kann beispielsweise eine Mischvorrichtung zur Herstellung individueller Haarbehandlungsmittel umfassen, eine separate Anzeigevorrichtung, beispielsweise in Form eines "intelligenten Spiegels, eine zentrale Steuerungssoftware, welche auf einer Datenverarbeitungsvorrichtung, beispielsweise einem Tablet, aufgespielt ist und die einzelnen Geräte und Datenflüsse steuert.

In verschiedenen Ausführungsbeispielen können von der Haarzustands-Ermittlungsvorrichtung als Teil eines Ecosystems erfasste Daten sowie ggf. weitere von einem Fachmann, zum Beispiel einem Friseur, über eine App oder eine andere geeignete Software eingegebene Daten, beispielsweise betreffend eine Optik und/oder eine Haptik von Haar mindestens eines Nutzers, in einer "Cloud" verarbeitet werden, wobei insbesondere eine Bestimmung einer Haarschädigung verarbeitet werden kann.

In verschiedenen Ausführungsbeispielen kann anhand des ermittelten Haarzustands die konkrete Zusammensetzung eines Haarbehandlungsmittels, beispielsweise eines Shampoos, einer Kur oder eines Färbemittels, berechnet werden. In verschiedenen Ausführungsbeispielen kann die Zusammensetzung eines individuell auf den ermittelten Haarzustand des Nutzers angepasstes Haarbehandlungsmittels ermittelt und an die Mischvorrichtung weitergeleitet werden. In der Mischvorrichtung kann beispielsweise aus mehreren Basisformulierung das individuelle Haarbehandlungsmittel herstellt werden. In verschiedenen Ausführungsformen kann das Ecosystem mittels einer zentralen Datenverarbeitungsvorrichtung, beispielsweise eines Tablets, iPads usw., gesteuert werden. In verschiedenen Ausführungsbeispielen können parallel die Ergebnisse, beispielsweise ein Grad der Haarschädigung, ein Feuchtigkeitsgehalt, eine berechnete Haarfarbe, mittels einer Anzeigevorrichtung, beispielsweise mittels eines smarten Spiegels, einem Nutzer oder Kunden visualisiert werden.

In verschiedenen Ausführungsbeispielen kann mittels der von der Haarzustands-Ermittlungsvorrichtung erfassten Daten und/oder des ermittelten Haarzustands ein individuelles Haarbehandlungsmittel für einen Nutzer herstellbar sein.

Gemäß Anspruch 13 wird ein Verfahren zum Bereitstellen von Haarzustandsinformation definiert. Das Verfahren umfasst *inter alia* ein Bewegen einer Haarzustands-Ermittlungsvorrichtung gemäß einem der Ansprüche 1 bis 12 entlang eines Bereiches von Haar eines Nutzers in einem vordefinierten Abstand zu dem Haar, wobei die Haarzustands-Ermittlungsvorrichtung ferner einen Beschleunigungssensor und eine elektronische Schaltkreisvorrichtung umfasst.

In verschiedenen Ausführungsbeispielen kann das Verarbeiten mindestens eines aus dem Folgenden umfassen: Speichern der mittels mindestens eines Sensors erfassten Haarzustandsinformation in einer Speichervorrichtung bei einem Erfassen eines ersten Bewegungsmusters; Löschen mindestens einer in der Speichervorrichtung und/oder in der externen Speichervorrichtung gespeicherten Haarzustandsinformation bei einem Erfassen eines zweiten Bewegungsmusters; Übermitteln der Haarzustandsinformation an eine externe Datenverarbeitungsvorrichtung und/oder an eine externe Speichervorrichtung bei einem Erfassen eines dritten Bewegungsmusters; Anzeigen von Information basierend auf der Haarzustandsinformation auf einer Anzeigevorrichtung der Vorrichtung und/oder auf einer Anzeigevorrichtung der externen Datenverarbeitungsvorrichtung bei einem Erfassen eines vierten Bewegungsmusters.

In verschiedenen Ausführungsbeispielen kann das Verfahren ferner umfassen: Durchführen des Erfassens der Haarfarbe zu einer ersten Zeit und Durchführen des Erfassens des Haarschädigungsgrades zu einer zweiten Zeit, wobei die erste Zeit und die zweite Zeit in einem vordefinierten Zeitbereich liegen.

In verschiedenen Ausführungsbeispielen kann anders ausgedrückt das Durchführen des Erfassens der Haarfarbe und das Durchführen des Erfassens des Haarschädigungsgrades annähernd gleichzeitig durchgeführt werden. In verschiedenen Ausführungsbeispielen kann dies ermöglichen, dass ein Datensatz ein Haarfarbwert (erfasst mittels des Farbsensors), ein Haarschädigungsgradwert (erfasst mittels des Haarzustandssensors) und eine Position im Raum (erfasst mittels des Beschleunigungssensors) umfassen kann. In verschiedenen Ausführungsbeispielen kann es ermöglicht werden, einen mehrdimensionalen Datensatz mittels der Haarzustands-Ermittlungsvorrichtung zur Weiterverarbeitung z.B. zum Anzeigen auf einer Anzeigevorrichtung bereitzustellen.

In verschiedenen Ausführungsbeispielen kann das Erfassen des Bewegungsmusters umfassen: Vergleichen der erfassten Bewegung mit einem vordefinierten Bewegungsmuster mittels einer elektronischen Schaltkreisvorrichtung; und Zuordnen der Bewegung zu einem vordefinierten Bewegungsmuster mittels der Schaltkreisvorrichtung.

In verschiedenen Ausführungsbeispielen kann das Verfahren ferner umfassen: Kalibieren zumindest eines Sensors der Mehrzahl von Sensoren mittels eines Kalibriermittels, wobei die Haarzustands-Ermittlungsvorrichtung das Kalibriermittel umfasst, wobei der mindestens eine Sensor der Mehrzahl von Sensoren gegenüberliegend zu dem Kalibriermittel positioniert ist, und wobei eine Sensorhauptmessrichtung des mindestens einen Sensors der Mehrzahl von Sensoren in einem vorbestimmten Winkel zu dem Kalibriermittel positioniert ist, und wobei das Kalibriermittel ein Keramikmaterial umfasst.

In verschiedenen Ausführungsbeispielen kann das Kalibrieren ferner umfassen: Zusammenführen eines ersten Bereichs und eines zweiten Bereichs der Haarzustands-Ermittlungsvorrichtung, derart, dass sich der erste Bereich und der zweite Bereich in einem vordefinierten Abstand einander gegenüberliegen, wobei in dem ersten Bereich der erste Sensor und der zweite Sensor positioniert sind und in dem zweiten Bereich das Kalibriermittel positioniert ist; und während des Zusammenführens Durchführen des Kalibrierens mindestens eines Sensors der Mehrzahl von Sensoren.

In verschiedenen Ausführungsbeispielen kann eine Haarzustands-Ermittlungsvorrichtung, welche sich beispielsweise in einem Ruhezustand (beispielsweise wird nur der Bewegungssensor und die Schaltkreisvorrichtung mit Energie versorgt) befindet, von einem Nutzer mit einer Hand aufgenommen werden. In verschiedenen Ausführungsbeispielen kann die Haarzustands-Ermittlungsvorrichtung mittels mindestens eines Beschleunigungssensors eine Bewegungsänderung der Haarzustands-Ermittlungsvorrichtung erfassen und der erfassten Bewegung entsprechende Sensorausgangsmessdaten an eine elektronische Schaltkreisvorrichtung drahtlos oder drahtgebunden übermitteln. In verschiedenen Ausführungsbeispielen kann die Schaltkreisvorrichtung die empfangenen Messdaten mit bekannten Bewegungsmessdaten vergleichen und mindestens einem bekannten Bewegungsmessdatensatz zuordnen. In verschiedenen Ausführungsbeispielen kann die Schaltkreisvorrichtung ein Aufwecken der sich im Schlafmodus befindenden Haarzustands-Ermittlungsvorrichtung in einen Betriebsmodus (beispielsweise wird neben dem Bewegungssensor und der Schaltkreisvorrichtung ferner mindestens eine Lichtquelle und mindestens ein Farbsensor und ein Haarzustandssensor mit Energie aus einem Akku versorgt) bewirken. In verschiedenen Ausführungsbeispielen kann der Nutzer vor dem Durchführen einer Messreihe die Haarzustands-Ermittlungsvorrichtung nach einem vordefinierte Bewegungsmuster bewegen, um einen Betriebszustand der Haarzustands-Ermittlungsvorrichtung zu erzielen, beispielsweise ein "Senkrechthalten der Haarzustands-Ermittlungsvorrichtung" nach einem Herausnehmen der Haarzustands-Ermittlungsvorrichtung aus einer externen Ladestation, ein "Senkrechthalten der Haarzustands-Ermittlungsvorrichtung und Schütteln", um eine neue Messreihe zu beginnen, ein "Ausschütteln der Haarzustands-Ermittlungsvorrichtung", um eine letzte Messung zu löschen usw.

In verschiedenen Ausführungsbeispielen kann die Haarzustands-Ermittlungsvorrichtung weitere Bewegungsmuster des Nutzers erfassen und entsprechende Befehle abarbeiten. In verschiedenen Ausführungsbeispielen kann die Haarzustands-Ermittlungsvorrichtung eingerichtet sein, dass sie von dem Nutzer neu bereitgestellte Bewegungsmuster, d.h. Bewegungen, bei der Nutzer seinen Arm mit der Haarzustands-Ermittlungsvorrichtung vordefiniert bewegt bzw. vordefiniert wiederholend bewegt, abspeichert und bestimmten Aktionen bzw. Messabläufe, die von der Haarzustands-Ermittlungsvorrichtung ausgeführt werden können, zugeordnet werden können.

In verschiedenen Ausführungsbeispielen kann mittels eines "Senkrechthaltens und Schüttelns der Haarzustands-Ermittlungsvorrichtung" vor der Durchführung einer neuen Messreihe die Durchführung der neuen Messreihe aktiviert werden. In verschiedenen Ausführungsbeispielen kann die Haarzustands-Ermittlungsvorrichtung derart parametriert sein, dass sie für die Aufnahme einer neuen Messreihe bereitsteht, beispielsweise der Farbsensor und/oder der Haarzustandssensor und/oder mindestens eine Lichtquelle an der Haarzustands-Ermittlungsvorrichtung aktiviert wird. In verschiedenen Ausführungsbeispielen kann der Nutzer die Haarzustands-Ermittlungsvorrichtung derart an Haar eines Verbrauchers bringen bzw. anlegen bzw. bewegen, dass sich das Haar zwischen einem ersten Bereich, der beispielsweise mindestens einen Sensor und/oder eine Lichtquelle umfassen kann, und einem zweiten Bereich, der zumindest einem Träger und/oder ein Kalibriermittel umfassen kann, befinden kann und mittels Handkraft des Nutzers zangenförmig von dem ersten und dem zweiten Bereich gegriffen werden kann, ohne eine Haarbeschädigung hervorzurufen.

In verschiedenen Ausführungsbeispielen kann mittels der Haarzustands-Ermittlungsvorrichtung an einer Stelle eine Haarzustandsmessung durchgeführt werden und die von dem Farbsensor und/oder dem Haarzustandssensor erfassten Messdaten an die Schaltkreisvorrichtung übermittelt werden.

In verschiedenen Ausführungsbeispielen kann mittels der Haarzustands-Ermittlungsvorrichtung eine Messreihe an sich ändernder Position durchgeführt werden, beispielsweise durch ein Bewegen der Haarzustands-Ermittlungsvorrichtung durch den Nutzer vom Haaransatz hin zu den Haarspitzen. In verschiedenen Ausführungsbeispielen kann die Schaltkreisvorrichtung die von den jeweiligen Sensoren empfangenen Messdaten bzw. Messreihen selbst auswerten oder zur Auswertung an eine externe Datenverarbeitungsvorrichtung drahtlos oder drahtgebunden übermitteln. In verschiedenen Ausführungsbeispielen kann eine Auswertung der erfassten Daten beispielsweise ein grafisches Darstellen von Haarzustandsinformation auf einer Anzeigevorrichtung der Haarzustands-Ermittlungsvorrichtung und/oder einer externen Datenverarbeitungsvorrichtung umfassen. In verschiedenen Ausführungsbeispielen können die erfassten Messdaten auch in einer Speichervorrichtung der Haarzustands-Ermittlungsvorrichtung und/oder der externen Datenverarbeitungsvorrichtung gespeichert werden.

In verschiedenen Ausführungsbeispielen kann der Nutzer der Haarzustands-Ermittlungsvorrichtung ein Speichern der erfassten Messdaten herbeiführen, indem er die Haarzustands-Ermittlungsvorrichtung nach einem vordefinierte Bewegungsmuster bewegt, um die Haarzustands-Ermittlungsvorrichtung in einem Speichermodus zu versetzen, beispielsweise mittels einer "Schnellen Bewegung von links nach rechts und wieder von rechts nach links" der Haarzustands-Ermittlungsvorrichtung vor der Durchführung einer neuen Messreihe.

In verschiedenen Ausführungsbeispielen kann nach einer erfolgreichen Durchführung mindestens einer Aufnahme von Sensormesswerten die Haarzustands-Ermittlungsvorrichtung mittels beispielsweise eines "Ausschüttelns" in einen Standby-Modus versetz werden, indem beispielsweise der Farbsensor und der Haarzustandssensor nicht weiter mit Energie versorgt werden können und nur noch der Beschleunigungssensor und die Schaltkreisvorrichtung mit Energie versorgt werden können, beispielsweise wenn sich die Haarzustands-Ermittlungsvorrichtung in einer Ladestation befindet.

In verschiedenen Ausführungsbeispielen kann es ermöglicht werden, dass eine Messreihe aus Haarfarbtönen und Haarschädigungsgraden und einer Position, bei der mindestens eine Haarfarbe und mindestens ein Haarschädigungsgrad von Haaren eines Verbrauchers erfasst worden sind, zur weiteren Datennachverarbeitung bereitgestellt werden können.

In verschiedenen Ausführungsbeispielen kann mittels der Haarzustands-Ermittlungsvorrichtung ermöglicht werden, dass bei einem wiederholten Durchführen einer Mehrzahl von Messreihen an Haar eines gleichbleibenden Verbrauchers nach einer vordefinierten Zeit die Messreihenergebnisse verglichen werden können, um beispielsweise einen Fortschritt in Bezug auf eine Minimierung eines Haarschädigungsgrads von Haar des Verbrauchers durch Anwendung geeigneter Haarpflegemittel verfolgen bzw. feststellen zu können.

In verschiedenen Ausführungsbeispielen kann die Vorrichtung mit einem Speicher zum Verwalten von Haarzustandsinformation über eine Datenverbindung Information, beispielsweise Messwerte betreffen eine Haarfarbe und/oder einen Haarschädigungsgrad, austauschen. Der Speicher kann ein Großdatenspeicher, beispielsweise ein Datenserver, ein Cloud-Server, einer Analyseplattform wie beispielsweise der Konstant-Information-Miner-Server (KNIME-Server) oder ein Big-Data-KNIME-Server sein. Mittels des Einsatzes eines KNIME-Servers können systematische Anwendungen statistischer Methoden auf große Datenmengen realisiert werden, wobei die großen Datenmengen, auch bezeichnet als "big data" bzw. Massendaten, nicht mit manuellen oder herkömmlichen Datenverarbeitungsmethoden auswertbar sein können. Unter Verwendung eines KNIME-Servers kann beispielsweise mittels einer grafischen Benutzeroberfläche ein einfaches und schnelles Aneinandersetzen von Modulen für die Datenvorverarbeitung von Nutzerdaten realisiert werden. Mittels einer Anwendung eines KNIME-Servers und einer big data können beispielsweise neue Querverbindungen und Trends im Bereich der Pflegeproduktindustrie ermittelt werden.

Die hierin beschriebenen Ausgestaltungen und genannten Vorteile beziehen sich auf die Vorrichtung nach Anspruch 1 und das Verfahren nach Anspruch 13.

Ausführungsbeispiele der Erfindung sind in den Figuren dargestellt und werden im Folgenden näher erläutert.

Es zeigen
- Fig. 1: eine schematische Seitenansicht einer Haarzustands-Ermittlungsvorrichtung zum Bereitstellen von Haarzustandsinformation gemäß verschiedenen Ausführungsbeispielen;
- Fig. 2: eine schematische Darstellung einer Haarzustands-Ermittlungsvorrichtung zum Bereitstellen von Haarzustandsinformation gemäß verschiedenen Ausführungsbeispielen;
- Figs. 3a, 3b, 3c: eine beispielhafte Bedienung einer Haarzustands-Ermittlungsvorrichtung zum Bereitstellen von Haarzustandsinformation gemäß verschiedenen Ausführungsbeispielen; und
- Fig. 4: ein beispielhaftes Ablaufdiagramm eines Verfahrens zum Bereitstellen von Haarzustandsinformation gemäß verschiedenen Ausführungsbeispielen.

In der folgenden ausführlichen Beschreibung wird auf die beigefügten Zeichnungen Bezug genommen, die Teil der vorliegenden Anmeldung bilden und in denen zur Veranschaulichung spezifische Ausführungsbeispiele gezeigt sind, in denen die Erfindung ausgeübt werden kann.

In dieser Hinsicht wird eine Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsbeispielen in einer Anzahl verschiedener Orientierungen positioniert werden können, dient die Richtungsterminologie zur Veranschaulichung und ist auf keinerlei Weise einschränkend. Es versteht sich von selbst, dass andere Ausführungsbeispiele benutzt und strukturelle oder logische Änderungen vorgenommen werden können, ohne von dem Schutzumfang der vorliegenden Erfindung abzuweichen.

Ferner versteht sich von selbst, dass die Merkmale der hierin beschriebenen verschiedenen beispielhaften Ausführungsbeispiele miteinander kombiniert werden können, sofern nicht spezifisch anders angegeben.

Die folgende ausführliche Beschreibung ist deshalb nicht in einschränkendem Sinne aufzufassen, und der Schutzumfang der vorliegenden Erfindung wird durch die angefügten Ansprüche definiert.

Unter einem digitalen Bild kann hierin ein Datenpaket verstanden werden, welches von einem Datenverarbeitungssystem als zweidimensionale (flächige) Anordnung von Bildpunkten darstellbar ist, beispielsweise in einem Koordinatensystem, welches eine x-Achse und eine y-Achse aufweist, wobei jedem Bildpunkt zumindest eine Farbinformation zugeordnet ist, welche beispielsweise als Farbe eines Pixels eines Monitors oder eines gedruckten Punktes eines ausgedruckten Bilds darstellbar ist. Dabei kann das digitale Bild beispielsweise ein mit einer Digitalkamera aufgenommenes Foto oder ein Einzelbild einer mit einer digitalen Kamera aufgenommenen Videosequenz sein.

Unter einer "Farbe" kann hierin ein Zusammenwirken eines Farbtons (d.h. eines spektralen Farbeindrucks, auch als Buntton bezeichnet, was als das verstanden werden kann, was als die "eigentliche Farbe" angesehen wird), einer Farbintensität (d.h. wie intensiv die Farbe erscheint, z.B. verglichen mit einem neutralen Grau, was auch als Sättigung, Farbsättigung, Buntheit, Chromatizität oder Farbtiefe bezeichnet wird) und einer Helligkeit (d.h. wie hell oder dunkel die Farbe erscheint) verstanden werden.

In verschiedenen Ausführungsbeispielen kann die Farbinformation beispielsweise eine Parametrisierung in einem bekannten Farbraum aufweisen, beispielsweise in einem L*a*b*-Farbraum (wobei L* die Helligkeit einer Farbe angibt, a* den Grün- und Rotanteil und b* den Blau- und Gelbanteil der Farbe), in einem RGB-Farbraum durch Farbanteile in Rot, Grün und Blau, in einem CMYK-Farbraum durch Farbanteile in Cyan, Magenta, Gelb und Schwarz, oder in einem beliebigen anderen Farbraum.

Unter dem Begriff "Farbton" kann hierin ein Farbwert bzw. der spektrale Farbeindruck einer Farbe verstanden werden, unabhängig davon, wie dieser parametrisiert sein kann, beispielsweise als ein Punkt in einem zweidimensionalen Farbraum (z.B. a*b* des L*a*b*-Systems) oder ein Verhältnis von Farbanteilen (wie z.B. beim RGB-Farbraum oder beim CMYK-Farbraum).

In verschiedenen Ausführungsbeispielen kann ein Farbraum, dem die Farbinformation (Haarfarbinformation und Bildfarbinformation) entstammt, so beschaffen sein, dass eine ermittelte oder dargestellte Farbe unabhängig von einem Medium ist, durch welches die Farbe ermittelt oder dargestellt wird (z.B. Bildschirm, Drucker, Scanner, menschliches Auge, usw.). Der Farbraum kann beispielsweise ein L*a*b*-Farbraum sein, die Farbinformation ein beispielsweise mittels a* und b* parametrisierter Farbton. Die einheitliche Darstellung in dem mediumunabhängigen Farbraum ermöglicht es, ein realitätsnahes zu erwartendes Färbeergebnis zu präsentieren.

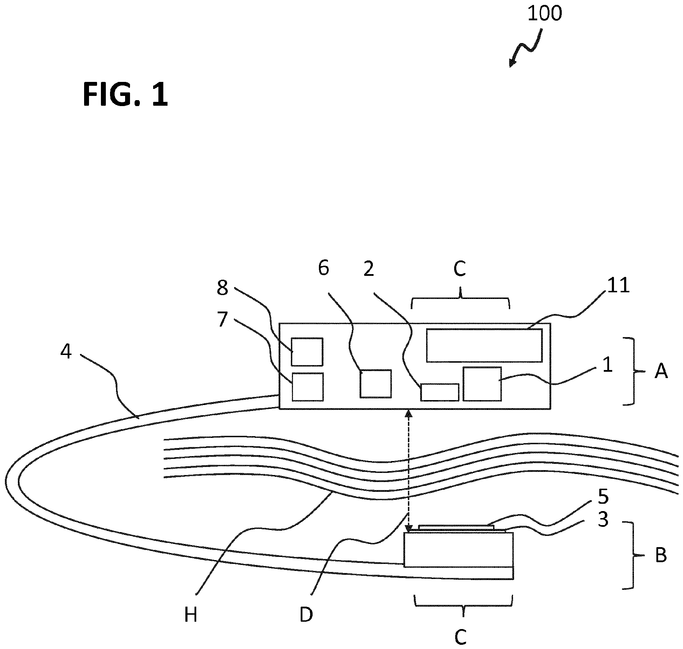

Figur 1 ist eine schematische Seitenansicht einer Haarzustands-Ermittlungsvorrichtung zum Bereitstellen von Haarzustandsinformation gemäß verschiedenen Ausführungsbeispielen.

In verschiedenen Beispielen umfasst die Haarzustands-Ermittlungsvorrichtung *inter alia* mindestens einen optischen Sensor 1, und gemäß der Erfindung einen Farbsensor 1a und einen Haarzustandssensor 1b, in einem ersten Bereich A . In verschiedenen Ausführungsbeispielen kann der erste Bereich A ferner einen Beschleunigungssensors 6, eine elektronische Schaltkreisvorrichtung 7, eine Speichervorrichtung 8 und optional eine Anzeigevorrichtung 11 umfassen. In verschiedenen Ausführungsbeispielen kann die Anzeigevorrichtung 11 an einer Oberseite der Haarzustands-Ermittlungsvorrichtung angeordnet sein.

In verschiedenen Ausführungsbeispielen kann der erste Bereich A ferner einen haptischen Signalgeber (Vibrator) umfassen.

Der erste Bereich A Z ist über ein Verbindungselement 4 mit einem zweiten Bereich B gekoppelt .

In verschiedenen Ausführungsbeispielen kann das Verbindungselement 4 einen U-förmigen und elastischen Federstahl umfassen. In verschiedenen Ausführungsbeispielen kann der zweite Bereich B einen Träger 3 und ein Kalibriermittel 5 umfassen. In verschiedenen Ausführungsbeispielen können der erste Bereich A und der zweite Bereich B jeweils an Endbereichen C des Verbindungselements 4 positioniert sein. In verschiedenen Ausführungsbeispielen kann eine untere Oberfläche des ersten Bereichs A einen vordefinierten Abstand D, beispielsweise in einem Bereich von etwa 0 mm bis etwa 200 mm, des zweiten Bereichs B aufweisen. In verschiedenen Ausführungsbeispielen kann zwischen dem ersten Bereich A und dem zweiten Bereich B Haar H eines Verbrauchers positioniert sein. In verschiedenen Ausführungsbeispielen kann die Lichtquelle 2 eingerichtet sein, während einer Messung von Haarzustandsinformation, beispielsweise einer Haarfarbe und eines Haarschädigungsgrads, den gegenüberliegenden Träger 3 und/oder das gegenüberliegende Kalibriermittel 5 zu beleuchten. In verschiedenen Ausführungsbeispielen kann mittels des Beschleunigungssensors 6 eine Bewegung bzw. ein Bewegungsmuster 6 erfasst und mit einer elektronischen Schaltkreisvorrichtung 7 mittels eines Vergleichs der erfassten Messdaten mit mindestens einem bekannten Bewegungsmuster ermittelt werden. In verschiedenen Ausführungsbeispielen kann mittels der Speichervorrichtung 8 mindestens ein erfasster Sensormessdatensatz archiviert bzw. abgespeichert werden. In verschiedenen Ausführungsbeispielen kann mittels der Anzeigevorrichtung 11 mindestens eine Haarzustandsinformation eines Verbrauchers und/oder einem Nutzer der Haarzustands-Ermittlungsvorrichtung dargestellt werden.

In verschiedenen Ausführungsbeispielen kann eine Haarzustands-Ermittlungsvorrichtung, welche sich beispielsweise in einem Standby-Modus (beispielsweise wird im Standby-Modus nur der Bewegungssensor und die Schaltkreisvorrichtung mit Energie versorgt) befindet, von einem Nutzer, beispielsweise einem Friseur oder einer Friseurin, mit einer Hand, d.h. einhändig, aufgenommen werden. In verschiedenen Ausführungsbeispielen kann die Haarzustands-Ermittlungsvorrichtung mittels mindestens eines Beschleunigungssensors 6 eine Bewegungsänderung der Haarzustands-Ermittlungsvorrichtung im Raum erfassen und einer erfassten Bewegung entsprechende Sensorausgangsmessdaten an eine elektronische Schaltkreisvorrichtung 7 drahtlos oder drahtgebunden übermitteln. In verschiedenen Ausführungsbeispielen kann die Schaltkreisvorrichtung 7 die empfangenen Messdaten mit bekannten Bewegungsmessdaten vergleichen und mindestens einem bekannten Bewegungsmessdatensatz zuordnen.

In verschiedenen Ausführungsbeispielen kann die Schaltkreisvorrichtung 7 ein Aufwecken der sich im Schlafmodus befindenden Haarzustands-Ermittlungsvorrichtung in einen Betriebsmodus (beispielsweise wird im Betriebsmodus neben dem Bewegungssensor 6 und der Schaltkreisvorrichtung 7 ferner mindestens eine Lichtquelle 2 und mindestens ein Farbsensor 1a und ein Haarzustandssensor 1b beispielsweise mit Energie aus einem Akku versorgt) bewirken.

In verschiedenen Ausführungsbeispielen kann der Nutzer vor dem Durchführen einer Messreihe die Haarzustands-Ermittlungsvorrichtung nach einem vordefinierte Bewegungsmuster bewegen, um ein Versetzen der Haar-Zustands-Ermittlungsvorrichtung in einen Betriebszustand zu erzielen, beispielsweise kann ein "Senkrechthalten der Haarzustands-Ermittlungsvorrichtung" nach einem Herausnehmen der Haarzustands-Ermittlungsvorrichtung aus einer externen Ladestation ein Aktivieren der Haarzustands-Ermittlungsvorrichtung bewirken, ein "Senkrechthalten der Haarzustands-Ermittlungsvorrichtung und Schütteln" kann verwendet werden, um eine neue Aufnahme mindestens einer Messreihe zu beginnen, ein "Ausschütteln der Haarzustands-Ermittlungsvorrichtung" kann verwendet werden, um eine letzte Messung und/oder Messreihe beispielsweise aus der Speichervorrichtung 7 zu löschen usw.

In verschiedenen Ausführungsbeispielen kann die Haarzustands-Ermittlungsvorrichtung noch weitere Bewegungsmuster des Nutzers erfassen und daraufhin entsprechende Befehle abarbeiten. In verschiedenen Ausführungsbeispielen kann die Haarzustands-Ermittlungsvorrichtung eingerichtet sein, dass sie von dem Nutzer neu bereitgestellte Bewegungsmuster, d.h. Bewegungen, bei denen der Nutzer seinen Arm zusammen mit der Haarzustands-Ermittlungsvorrichtung vordefiniert bewegt bzw. vordefiniert wiederholend bewegt, erlernen kann und abspeichern kann und bestimmten Aktionen, die von der Haarzustands-Ermittlungsvorrichtung ausgeführt werden können, zuordnen kann.

In verschiedenen Ausführungsbeispielen kann mittels eines "Senkrechthaltens und Schüttelns" der Haarzustands-Ermittlungsvorrichtung vor der Durchführung einer neuen Messreihe die Durchführung der neuen Messreihe aktiviert werden. In verschiedenen Ausführungsbeispielen kann die Haarzustands-Ermittlungsvorrichtung dann derart parametriert sein, dass sie für die Aufnahme mindestens einer neuen Messreihe bereit ist, und beispielsweise der Farbsensor 1a und/oder der Haarzustandssensor 1b und/oder mindestens eine Lichtquelle 2 an der Haarzustands-Ermittlungsvorrichtung aktiviert werden. In verschiedenen Ausführungsbeispielen kann der Nutzer die Haarzustands-Ermittlungsvorrichtung derart an Haar H eines Verbrauchers anbringen bzw. anlegen bzw. bewegen, dass sich das Haar zwischen einem ersten Bereich A, der beispielsweise mindestens einen Sensor 1 und/oder eine Lichtquelle 2 umfassen kann, und einem zweiten Bereich B, der zumindest einen Träger 3 und/oder ein Kalibriermittel 5 umfassen kann, befindet und mittels Handkraft des Nutzers zangenförmig von dem ersten Bereich A und dem zweiten Bereich B gegriffen werden kann, ohne eine Haarbeschädigung hervorzurufen.

In verschiedenen Ausführungsbeispielen kann, um eine Mehrzahl von Haarzustandsmesswerten mittels der Vorrichtung zu erfassen, der erste Bereich A und der zweite Bereich B mittels Muskelkraft des Nutzers aufeinander zu bewegt werden, während sich zwischen dem erste Bereich A und dem zweiten Bereich B das zu vermessende Haar H eines Verbrauchers befindet.

In verschiedenen Ausführungsbeispielen kann der erste Sensor 1a, der Farbsensor, und/oder der zweite Sensor 1b, der NIR-Sensor, bei einem Kalibriervorgang mittels des Kalibriermittels 5 nach einem Aktivieren des Farbsensors und/oder des NIR-Sensors und einer Lichtquelle 2 in einen Kalibriermodus gebracht werden. In verschiedenen Ausführungsbeispielen kann dann das Kalibriermittel 5 bis auf einen vordefinierten Abstand, beispielsweise auf einen Abstand von etwa 0 mm bis etwa 10 mm, hin zu dem gegenüberliegenden Farbsensor 1a und/oder dem NIR-Sensor 1b mittels Muskelkraft verfahren werden. In verschiedenen Ausführungsbeispielen kann beispielsweise bei einem Kalibriervorgang des NIR-Sensors das Kalibriermittel direkt vor oder direkt an eine Hauptmessoberfläche des NIR-Sensors positioniert werden. In verschiedenen Ausführungsbeispielen kann dann der Farbsensor 1a mittels der Lichtquelle 2 und/oder der NIR-Sensor 1b mittels einer Infrarotquelle (IR-Quelle) eine den Sensoren 1 gegenüberliegende Oberfläche des Kalibriermittels 5, beispielsweise einer Keramik, beleuchten und eine Reflektion (und dadurch auch die Absorption) der reflektierten Lichtstrahlen und/oder Infrarotstrahlen erfassen bzw. messen. In verschiedenen Ausführungsbeispielen kann der Farbsensor 1a und/oder der NIR-Sensor 1b dann ein internes Kalibrierprofil für alle weiteren Messungen generieren.

In verschiedenen Ausführungsbeispielen kann mittels der Haarzustands-Ermittlungsvorrichtung an einer Stelle der Haare eine Haarzustandsmessung durchgeführt werden und die von dem Farbsensor 1a und/oder dem Haarzustandssensor 1b erfassten Messdaten an die Schaltkreisvorrichtung 7 übermittelt werden.

In verschiedenen Ausführungsbeispielen kann der erste Sensor 1a, der Farbsensor, und/oder der zweite Sensor 1b, der NIR-Sensor, bei eingeschalteter Lichtquelle 2 und/oder Infrarotquelle Licht emittieren, das auf Haar eines Verbrauchers fallen kann, wobei das emittierte Licht mittels des Haars bzw. am Haar zurück reflektiert wird. In verschiedenen Ausführungsbeispielen können die reflektierten Lichtstrahlen und/oder Infrarotstrahlen von den Sensoren 1 erfasst bzw. gemessen werden und beispielsweise in der Speichervorrichtung 8 gespeichert werden. In verschiedenen Ausführungsbeispielen kann mittels des Farbsensors 1a eine mehrkanalige Farbmessung mit Transformation in einen Darstellungsraum, beispielsweise CIE LAB, durchgeführt werden.

In verschiedenen Ausführungsbeispielen kann mittels der Haarzustands-Ermittlungsvorrichtung eine Messreihe an sich ändernden Positionen durchgeführt werden, beispielsweise durch ein Bewegen der Haarzustands-Ermittlungsvorrichtung durch den Nutzer vom Haaransatz hin zu den Haarspitzen. In verschiedenen Ausführungsbeispielen kann die Schaltkreisvorrichtung 7 die von den jeweiligen Sensoren 1a, 1b empfangenen Messdaten bzw. Messreihen selbst auswerten oder zur Auswertung an mindestens eine externe Datenverarbeitungsvorrichtung 10 drahtlos oder drahtgebunden übermitteln. In verschiedenen Ausführungsbeispielen kann eine Auswertung der erfassten Daten beispielsweise ein grafisches Darstellen von Haarzustandsinformation auf einer Anzeigevorrichtung 11 der Haarzustands-Ermittlungsvorrichtung und/oder einer externen Datenverarbeitungsvorrichtung 12 umfassen. In verschiedenen Ausführungsbeispielen können die erfassten Messdaten auch in einer Speichervorrichtung 8 der Haarzustands-Ermittlungsvorrichtung und/oder der externen Datenverarbeitungsvorrichtung 10 gespeichert werden.

In verschiedenen Ausführungsbeispielen kann der Nutzer der Haarzustands-Ermittlungsvorrichtung ein Speichern der erfassten Messdaten herbeiführen, indem er die Haarzustands-Ermittlungsvorrichtung nach einem vordefinierte Bewegungsmuster bewegt, um die Haarzustands-Ermittlungsvorrichtung in einen Speichermodus zu versetzen, beispielsweise mittels einer "Schnellen Bewegung von links nach rechts und wieder von rechts nach links" der Haarzustands-Ermittlungsvorrichtung vor der Durchführung einer neuen Messreihe.

In verschiedenen Ausführungsbeispielen kann nach einer erfolgreichen Durchführung mindestens einer Aufnahme von Sensormesswerten die Haarzustands-Ermittlungsvorrichtung beispielsweise mittels eines "Ausschüttelns" in einen Standby-Modus versetz werden, indem beispielsweise der Farbsensor 1a und der Haarzustandssensor 1b und die Lichtquelle 2 und die Infrarotquelle nicht weiter mit Energie versorgt werden können und nur noch der Beschleunigungssensor 6 und die Schaltkreisvorrichtung 7 mit Energie versorgt werden können, beispielsweise wenn sich die Haarzustands-Ermittlungsvorrichtung in einer Ladestation befindet.

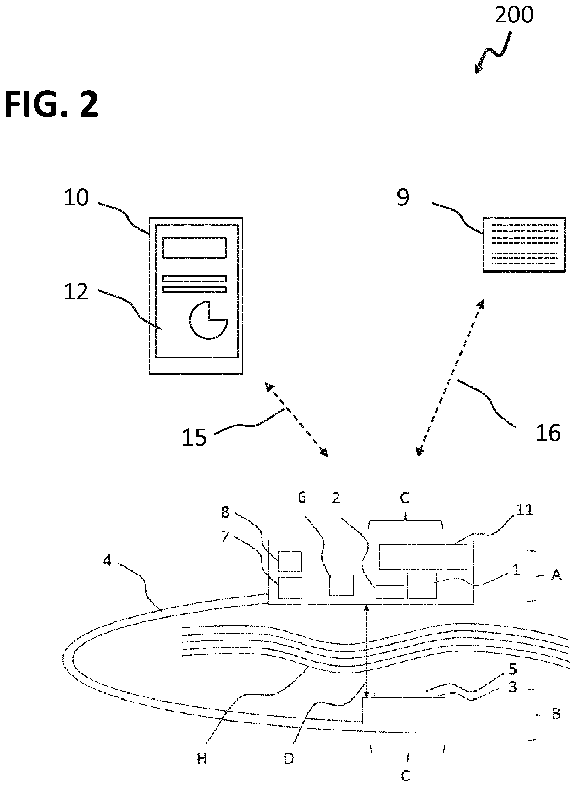

Figur 2 zeigt eine schematische Darstellung einer Vorrichtung zum Bereitstellen von Haarzustandsinformation gemäß verschiedenen Ausführungsbeispielen.

In verschiedenen Ausführungsbeispielen kann die Haarzustands-Ermittlungsvorrichtung über ein Datenübertragungsmodul verfügen mittels dessen erfasste Messdaten über eine Verbindung 15, beispielsweise eine Drahtlosverbindung, an mindestens eine externe Datenverarbeitungsvorrichtung 10, beispielsweise ein Smartphone oder Tablet, übermittelt werden können. In verschiedenen Ausführungsbeispielen können die an die externe Datenübertragungsvorrichtung 10 übermittelten Messdaten beispielsweise mittels einer Anzeigevorrichtung 12 grafisch dargestellt werden. In verschiedenen Ausführungsbeispielen kann die Haarzustands-Ermittlungsvorrichtung über ein Datenübertragungsmodul verfügen, mittels dessen erfasste Messdaten über eine Verbindung 16, beispielsweise eine Drahtlosverbindung, an mindestens eine externe Speichervorrichtung 9 übermittelt werden können. In verschiedenen Ausführungsbeispielen kann das Datenübertragungsmodul dasselbe sein und die Datenübertragung über die Verbindungen 15 und 16 gleichzeitig realisiert sein.

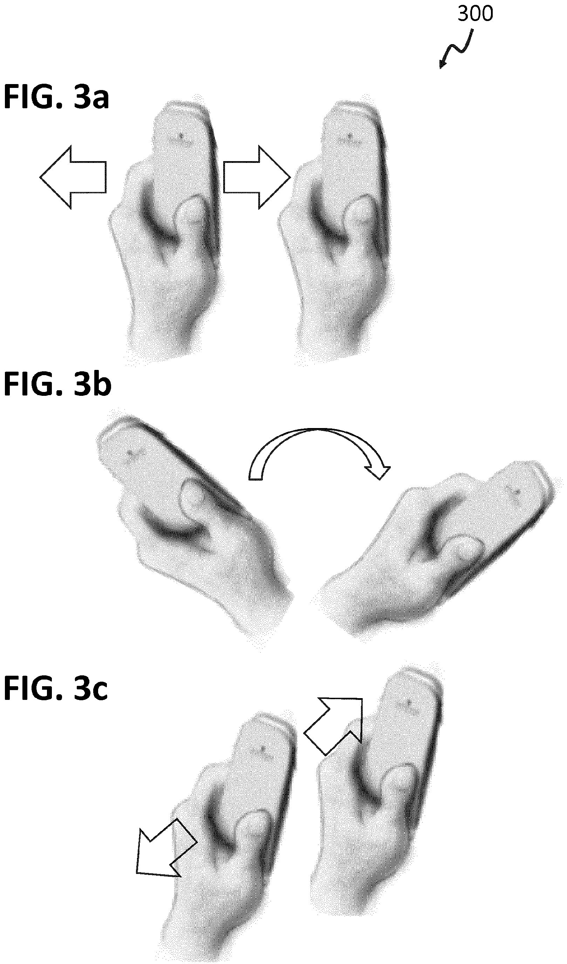

Figs. 3a, 3b, 3c veranschaulichen eine beispielhafte Bedienung einer Haarzustands-Ermittlungsvorrichtung zum Bereitstellen von Haarzustandsinformation durch einen Nutzer gemäß verschiedenen Ausführungsbeispielen.

In Fig. 3a ist ein Bewegungsmuster veranschaulicht, bei dem der Nutzer die Haarzustands-Ermittlungsvorrichtung beispielsweise zuerst senkrecht hält und dann horizontal von links nach rechts schüttelt. Dieses Bewegungsmuster kann beispielsweise von der Schaltkreisvorrichtung 7 erkannt bzw. registriert werden. Die Haarzustands-Ermittlungsvorrichtung kann daraufhin derart eingestellt werden, dass sie für eine Aufnahme einer neuen Messreihe bereitsteht.

In Fig. 3b ist ein Bewegungsmuster veranschaulicht, bei dem der Nutzer die Haarzustands-Ermittlungsvorrichtung beispielsweise um etwa eine Achse "ausschüttelt", um die Haarzustands-Ermittlungsvorrichtung zu veranlassen, zuvor erfasste Messdaten bzw. mindestens eine Messreihe zu löschen.

In Fig. 3c ist ein weiteres beispielhaftes Bewegungsmuster veranschaulicht, bei dem der Nutzer die Haarzustands-Ermittlungsvorrichtung beispielsweise veranlassen kann, eine Übermittlung von gemessenen Messdaten an eine externe Datenverarbeitungsvorrichtung 10 zu übermitteln.

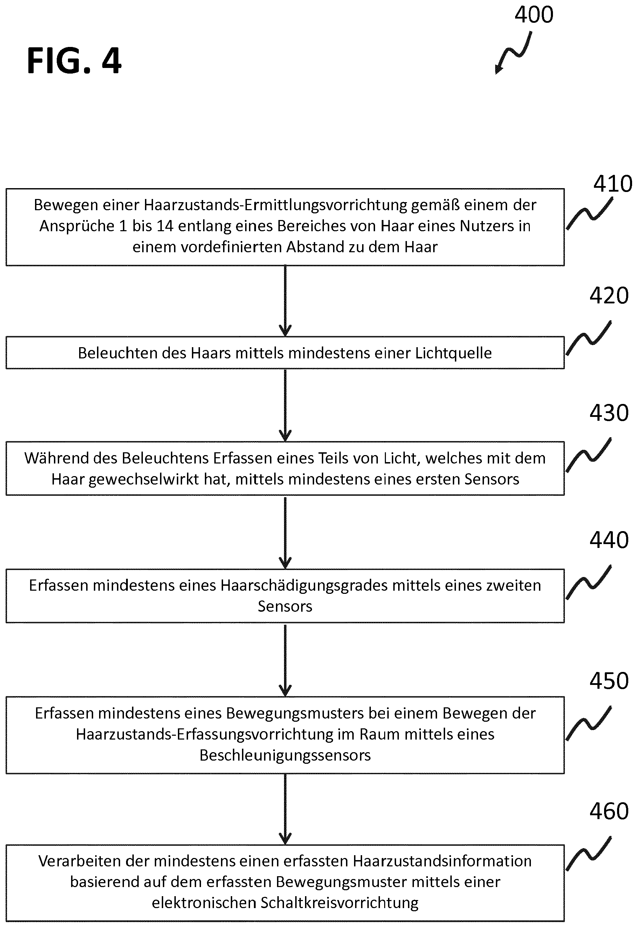

Fig. 4 zeigt ein beispielhaftes Ablaufdiagramm eines Verfahrens zum Bereitstellen von Haarzustandsinformation gemäß verschiedenen Ausführungsbeispielen.

Das Verfahren zum Bereitstellen von Haarzustandsinformation umfasst gemäß Anspruch 13 *inter alia* ein Bewegen einer Haarzustands-Ermittlungsvorrichtung gemäß einem der Ansprüche 1 bis 12 entlang eines Bereiches von Haar eines Nutzers in einem vordefinierten Abstand zu dem Haar, ein Beleuchten des Haars mittels mindestens einer Lichtquelle, während des Beleuchtens Erfassen eines Teils von Licht, welches mit dem Haar gewechselwirkt hat, mittels mindestens eines ersten Sensors, Erfassen mindestens eines Haarschädigungsgrades mittels eines zweiten Sensors, Erfassen mindestens eines Bewegungsmusters bei einem Bewegen der Haarzustands-Ermittlungsvorrichtung im Raum mittels eines Beschleunigungssensors; und ein Verarbeiten der mindestens einen erfassten Haarzustandsinformation basierend auf dem erfassten Bewegungsmuster mittels einer elektronischen Schaltkreisvorrichtung, umfassen.

Die hierin in Bezug auf die Haarzustands-Ermittlungsvorrichtung beschriebenen Eigenschaften und Vorteile beziehen sich selbstverständlich auch auf das hierin beschriebene Verfahren und umgekehrt.

## Patentansprüche

1. Haarzustands-Ermittlungsvorrichtung zum Bereitstellen von Haarzustandsinformation, aufweisend:
einen ersten Bereich (A) und einen zweiten Bereich (B), welche so eingerichtet sind, dass Haare eines Nutzers zwischen dem ersten Bereich (A) und dem zweiten Bereich (B) bewegbar sind,
wobei in dem ersten Bereich (A) mindestens eine Lichtquelle (2) angeordnet ist, und wobei in dem ersten Bereich mindestens ein optischer Sensor zum Erfassen von Licht der mindestens einen Lichtquelle (2) als die Haarzustandsinformation angeordnet ist,
wobei der zweite Bereich (B) mindestens einen Träger (3) umfasst, und
wobei der erste Bereich (A) und der zweite Bereich (B) mittels eines Verbindungselements (4) derart gekoppelt sind, dass sich der erste Bereich (A) und der zweite Bereich (B) derart gegenüberliegen, dass eine Sensorhauptmessrichtung des mindestens einen Sensors in einem vordefinierten Winkel zu einer Oberfläche des Trägers (3) ausgerichtet ist;
wobei der mindestens eine optische Sensor im ersten Bereich (A) einen Farbsensor (1a) zum Erfassen einer Haarfarbe des Nutzers umfasst;
**dadurch gekennzeichnet, dass** im ersten Bereich (A) ferner ein Haarzustandssensor (1b) zum Erfassen eines Haarschädigungsgrads des Nutzers umfasst ist,
wobei der Haarzustandssensor (1b) einen Nahinfrarotspektroskopie-Sensor umfasst, und
wobei der Haarzustandssensor eingerichtet ist, eine Haarschädigung als einen Gehalt an Cysteinsäure zu ermitteln.

2. Vorrichtung gemäß Anspruch 1, wobei das Verbindungselement (4) einen elastischen Werkstoff umfasst.

3. Vorrichtung gemäß einem der Ansprüche 1 oder 2, wobei das Verbindungselement (4) eine U-förmige Ausgestaltung aufweist und der erste Bereich (A) und der zweite Bereich (B) jeweils an einem Endbereich (C) der U-förmigen Ausgestaltung positioniert sind.

4. Vorrichtung gemäß einem der vorherigen Ansprüche, wobei auf einem dem ersten Bereich (A) zugewandten Bereich einer Oberfläche des Trägers (3) mindestens ein Kalibriermittel (5) angeordnet ist, wobei das Kalibriermittel (5) eingerichtet ist, zumindest den Sensor (1) der Vorrichtung zu kalibrieren.

5. Vorrichtung gemäß Anspruch 4, wobei das Kalibriermittel (5) ein Keramikmaterial umfasst oder ist.

6. Vorrichtung gemäß einem der vorherigen Ansprüche, wobei der Farbsensor (1a) ferner zum Erfassen einer Haarschädigung über UV-Licht mittels einer Absorption oder Reflektion geeignet ist, wobei der Farbsensor (1a) geeignet ist, zu einer ersten Zeit eine Haarfarbe des Nutzers zu erfassen und der Haarzustandssensor (1b) geeignet ist, zu einer zweiten Zeit einen Haarschädigungsgrad des Nutzers zu erfassen, und wobei die erste Zeit und die zweite Zeit in einem vordefinierten Zeitbereich liegen.

7. Vorrichtung gemäß einem der vorherigen Ansprüche, wobei der erste Bereich (A) oder der zweite Bereich (B) ferner einen Beschleunigungssensor (6) umfasst.

8. Vorrichtung gemäß Anspruch 7, wobei der Beschleunigungssensor (6) eingerichtet ist, einen Bewegungszustand der Vorrichtung im Raum zu erfassen.

9. Vorrichtung gemäß einem der vorherigen Ansprüche, wobei die Vorrichtung ferner eine elektronische Schaltkreisvorrichtung (7) umfasst.

10. Vorrichtung gemäß Anspruch 9,
wobei die Schaltkreisvorrichtung (7) zumindest eingerichtet ist, basierend auf zumindest einem mittels des Beschleunigungssensors (6) erfassten Bewegungszustand der Vorrichtung einen Betriebszustand der Vorrichtung zu steuern.

11. Vorrichtung gemäß Anspruch 10, wobei
die Schaltkreisvorrichtung (7) basierend auf dem jeweiligen erfassten Bewegungszustand ferner eingerichtet ist,
zumindest eine mittels des mindestens einen Sensors (1) erfasste Haarzustandsinformation in einer Speichervorrichtung (8) der Vorrichtung oder in einer externen Speichervorrichtung (9) zu speichern, und/oder
mindestens eine Haarzustandsinformation aus der Speichervorrichtung (8) der Vorrichtung oder aus der externen Speichervorrichtung (9) zu löschen, und/oder die Haarzustandsinformation an eine externe Datenverarbeitungsvorrichtung (10) zu übermitteln, und/oder
Information basierend auf der Haarzustandsinformation auf einer Anzeigevorrichtung (11) der Vorrichtung und/oder auf einer Anzeigevorrichtung (12) der externen Datenverarbeitungsvorrichtung (10) anzuzeigen.

12. Vorrichtung gemäß einem der Ansprüche 1 bis 11, wobei mittels des von der Haarzustands-Ermittlungsvorrichtung ermittelten Haarzustands ein individuelles Haarbehandlungsmittel für einen Nutzer herstellbar ist.

13. Verfahren zum Bereitstellen von Haarzustandsinformation, aufweisend:
• Bewegen einer Haarzustands-Ermittlungsvorrichtung gemäß einem der Ansprüche 1 bis 12 entlang eines Bereiches von Haar eines Nutzers in einem vordefinierten Abstand (D) zu dem Haar, wobei die Haarzustands-Ermittlungsvorrichtung ferner einen Beschleunigungssensor (6) und eine elektronische Schaltkreisvorrichtung (7) umfasst;
• Beleuchten des Haars mittels mindestens der Lichtquelle (2);
• Während des Beleuchtens Erfassen eines Teils von Licht, welches mit dem Haar (H) gewechselwirkt hat, mittels mindestens des Farbsensors (1a);
• Erfassen mindestens des Haarschädigungsgrades mittels des Haarzustandssensors (1b);
• Erfassen mindestens eines Bewegungsmusters bei einem Bewegen der Haarzustands-Ermittlungsvorrichtung im Raum mittels des Beschleunigungssensors (6); und
• Verarbeiten der mindestens einen erfassten Haarzustandsinformation basierend auf dem erfassten Bewegungsmuster mittels der elektronischen Schaltkreisvorrichtung (7).

14. Verfahren gemäß Anspruch 13, wobei das Verarbeiten mindestens eines aus dem Folgenden umfasst:
• Speichern der mittels mindestens eines Sensors (1a,1b) erfassten Haarzustandsinformation in einer Speichervorrichtung (8) bei einem Erfassen eines ersten Bewegungsmusters;
• Löschen mindestens einer in der Speichervorrichtung (8) und/oder in der externen Speichervorrichtung (9) gespeicherten Haarzustandsinformation bei einem Erfassen eines zweiten Bewegungsmusters;
• Übermitteln der Haarzustandsinformation an eine externe Datenverarbeitungsvorrichtung (10)und/oder an eine externe Speichervorrichtung (9) bei einem Erfassen eines dritten Bewegungsmusters; und
• Anzeigen von Information basierend auf der Haarzustandsinformation auf einer Anzeigevorrichtung (11) der Vorrichtung und/oder auf einer Anzeigevorrichtung (12) der externen Datenverarbeitungsvorrichtung (10) bei einem Erfassen eines vierten Bewegungsmusters.

15. Verfahren gemäß einem der Ansprüche 13 oder 14, wobei das Verfahren ferner umfasst:
• Durchführen des Erfassens der Haarfarbe zu einer ersten Zeit; und
• Durchführen des Erfassens des Haarschädigungsgrades zu einer zweiten Zeit, wobei die erste Zeit und die zweite Zeit in einem vordefinierten Zeitbereich liegen.

## Claims

1. A hair condition determination device for providing hair condition information, comprising:
a first region (A) and a second region (B) which are designed in such a way that a user's hair can be moved between the first region (A) and the second region (B),
at least one light source (2) being arranged in the first region (A), and
at least one optical sensor for detecting light from the at least one light source (2) as the hair condition information being arranged in the first region,
the second region (B) comprising at least one support (3), and
the first region (A) and the second region (B) being coupled by means of a connecting element (4) such that the first region (A) and the second region (B) are opposite one another such that a main sensor measuring direction of the at least one sensor is oriented at a predefined angle to a surface of the support (3);
the at least one optical sensor in the first region (A) comprising a color sensor (1a) for detecting a hair color of the user;
**characterized in that**, in the first region (A), a hair condition sensor (1b) for detecting a degree of damage to the user's hair is also comprised,
the hair condition sensor (1b) comprising a near-infrared spectroscopy sensor, and the hair condition sensor being designed to determine hair damage in the form of a content of cysteic acid.

2. The device according to claim 1, wherein the connecting element (4) comprises an elastic material.

3. The device according to one of claims 1 or 2, wherein the connecting element (4) has a U-shaped configuration, and the first region (A) and the second region (B) are each positioned at an end region (C) of the U-shaped configuration.

4. The device according to one of the preceding claims, wherein at least one calibration means (5) is arranged on a region of a surface of the support (3) facing the first region (A), wherein the calibration means (5) is designed to calibrate at least the sensor (1) of the device.

5. The device according to claim 4, wherein the calibration means (5) comprises or is a ceramic material.

6. The device according to one of the preceding claims, wherein the color sensor (1a) is also suitable for detecting hair damage via UV light by means of absorption or reflection,
wherein the color sensor (1a) is suitable for detecting a hair color of the user at a first time and the hair condition sensor (1b) is suitable for detecting a degree of damage to the user's hair at a second time, and
wherein the first time and the second time are within a predefined time range.

7. The device according to one of the preceding claims, wherein the first region (A) or the second region (B) further comprises an acceleration sensor (6).

8. The device according to claim 7, wherein the acceleration sensor (6) is designed to detect a movement state of the device in space.

9. The device according to one of the preceding claims, wherein the device further comprises an electronic circuit device (7).

10. The device according to claim 9,
wherein the circuit device (7) is at least designed to control an operating state of the device based on at least one movement state of the device detected by the acceleration sensor (6).

11. The device according to claim 10, wherein
the circuit device (7) is further designed, based on the particular detected movement state,
to store, in a memory device (8) of the device or in an external memory device (9), at least one item of hair condition information detected by means of the at least one sensor (1), and/or
to delete, from the memory device (8) of the device or from the external memory device (9), at least one item of hair condition information, and/or
to transmit the hair condition information to an external data processing device (10), and/or
to display, on a display device (11) of the device and/or on a display device (12) of the external data processing device (10), information based on the hair condition information.

12. The device according to one of claims 1 to 11, wherein an individual hair treatment agent can be produced for a user using the hair condition determined by the hair condition determination device.

13. A method for providing hair condition information, comprising:
• moving a hair condition determination device according to one of claims 1 to 12 along a region of a user's hair at a predefined distance (D) from the hair, wherein the hair condition determination device further comprises an acceleration sensor (6) and an electronic circuit device (7);
• illuminating the hair using at least the light source (2);
• during the illumination, detecting, by means of at least the color sensor (1a), a portion of light which has interacted with the hair (H);
• detecting, by means of the hair condition sensor (1b), at least the degree of hair damage;
• detecting, by means of the acceleration sensor (6), at least one movement pattern when the hair condition determination device is moved in space; and
• processing, by means of the electronic circuit device (7), the at least one item of detected hair condition information based on the detected movement pattern.

14. The method according to claim 13, wherein the processing comprises at least one of the following:
• when a first movement pattern is detected, storing, in a storage device (8), the hair condition information detected by means of at least one sensor (1a, 1b);
• when a second movement pattern is detected, deleting at least one item of hair condition information stored in the storage device (8) and/or in the external storage device (9);
• when a third movement pattern is detected, transmitting the hair condition information to an external data processing device (10) and/or to an external storage device (9); and
• when a fourth movement pattern is detected, displaying, on a display device (11) of the device and/or on a display device (12) of the external data processing device (10), information based on the hair condition information.

15. The method according to one of claims 13 or 14, wherein the method further comprises:
• performing the detection of the hair color at a first time; and
• performing the detection of the degree of hair damage at a second time, wherein the first time and the second time are within a predefined time range.

## Revendications

1. Dispositif de détermination de l'état des cheveux permettant de fournir des informations concernant l'état des cheveux, présentant :
une première zone (A) et une seconde zone (B) qui sont conçues de manière à ce que des cheveux d'un utilisateur peuvent être déplacés entre la première zone (A) et la seconde zone (B),
dans lequel au moins une source de lumière (2) est disposée dans la première zone (A), et
dans lequel au moins un capteur optique permettant de détecter de la lumière provenant de l'au moins une source de lumière (2) en tant qu'informations concernant l'état des cheveux est disposé dans la première zone,
dans lequel la seconde zone (B) comprend au moins un support (3), et
dans lequel la première zone (A) et la seconde zone (B) sont couplées au moyen d'un élément de liaison (4) de telle manière que la première zone (A) et la seconde zone (B) sont opposées l'une à l'autre de sorte qu'une direction de mesure principale de capteur de l'au moins un capteur est disposée selon un angle prédéfini par rapport à une surface du support (3) ;
dans lequel l'au moins un capteur optique dans la première zone (A) comprend un capteur de couleur (1a) permettant de détecter une couleur de cheveux de l'utilisateur ;
**caractérisé en ce que** la première zone (A) comprend en outre un capteur d'état des cheveux (1b) permettant de détecter un degré d'endommagement des cheveux de l'utilisateur,
dans lequel le capteur d'état des cheveux (1b) comprend un capteur de spectroscopie dans le proche infrarouge, et dans lequel le capteur d'état des cheveux est configuré pour déterminer un endommagement des cheveux en tant que teneur en acide cystéique.

2. Dispositif selon la revendication 1, dans lequel l'élément de liaison (4) comprend un matériau élastique.

3. Dispositif selon l'une des revendications 1 ou 2, dans lequel l'élément de liaison (4) présente une configuration en forme de U et la première zone (A) et la seconde zone (B) sont respectivement positionnées au niveau d'une zone d'extrémité (C) de la configuration en forme de U.

4. Dispositif selon l'une des revendications précédentes, dans lequel au moins un moyen de calibrage (5) est disposé sur une zone d'une surface du support (3) faisant face à la première zone (A), le moyen de calibrage (5) étant configuré pour calibrer au moins le capteur (1) du dispositif.

5. Dispositif selon la revendication 4, dans lequel le moyen de calibrage (5) comprend ou est un matériau céramique.

6. Dispositif selon l'une des revendications précédentes, dans lequel le capteur de couleur (1a) est en outre adapté pour détecter un endommagement des cheveux au moyen de la lumière UV par absorption ou réflexion,
dans lequel le capteur de couleur (1a) est adapté pour détecter une couleur de cheveux de l'utilisateur dans un premier temps et le capteur d'état des cheveux (1b) est adapté pour détecter un degré d'endommagement des cheveux de l'utilisateur dans un second temps, et
dans lequel le premier temps et le second temps se situent dans une plage de temps prédéfinie.

7. Dispositif selon l'une des revendications précédentes, dans lequel la première zone (A) ou la seconde zone (B) comprend en outre un capteur d'accélération (6).

8. Dispositif selon la revendication 7, dans lequel le capteur d'accélération (6) est configuré pour détecter un état de mouvement du dispositif dans l'espace.

9. Dispositif selon l'une des revendications précédentes, dans lequel le dispositif comprend en outre un dispositif de circuit (7) électronique.

10. Dispositif selon la revendication 9,
dans lequel le dispositif de circuit (7) est au moins configuré pour commander un état de fonctionnement du dispositif sur la base d'au moins un état de mouvement du dispositif détecté au moyen du capteur d'accélération (6).

11. Dispositif selon la revendication 10, dans lequel
le dispositif de circuit (7) est en outre configuré, sur la base de l'état de mouvement détecté respectif,
pour stocker au moins une information concernant l'état des cheveux détectée au moyen de l'au moins un capteur (1) dans un dispositif de stockage (8) du dispositif ou dans un dispositif de stockage externe (9), et/ou
pour supprimer au moins une information concernant l'état des cheveux du dispositif de stockage (8) du dispositif ou du dispositif de stockage externe (9), et/ou
pour transmettre l'information concernant l'état des cheveux à un dispositif de traitement de données externe (10), et/ou
pour afficher des informations sur la base de l'information concernant l'état des cheveux sur un dispositif d'affichage (11) du dispositif et/ou sur un dispositif d'affichage (12) du dispositif de traitement de données externe (10).

12. Dispositif selon l'une des revendications 1 à 11, dans lequel un agent de traitement des cheveux individuel peut être produit pour un utilisateur au moyen de l'état des cheveux déterminé par le dispositif de détermination de l'état des cheveux.

13. Procédé permettant de fournir des informations concernant l'état des cheveux, présentant :
• le déplacement d'un dispositif de détermination de l'état des cheveux selon l'une des revendications 1 à 12 le long d'une zone des cheveux d'un utilisateur à une distance prédéfinie (D) des cheveux, le dispositif de détermination de l'état des cheveux comprenant en outre un capteur d'accélération (6) et un dispositif de circuit (7) électronique ;
• l'éclairage des cheveux au moyen d'au moins la source de lumière (2) ;
• pendant l'éclairage, la détection d'une partie de la lumière qui a interagi avec les cheveux (H) au moyen d'au moins le capteur de couleur (1a) ;
• la détection d'au moins le degré d'endommagement des cheveux au moyen du capteur d'état des cheveux (1b) ;
• la détection d'au moins un modèle de mouvement lors d'un déplacement du dispositif de détermination de l'état des cheveux dans l'espace au moyen du capteur d'accélération (6) ; et
• le traitement de l'au moins une information concernant l'état des cheveux détectée sur la base du modèle de mouvement détecté au moyen du dispositif de circuit (7) électronique.

14. Procédé selon la revendication 13, dans lequel le traitement comprend :
• le stockage des informations concernant l'état des cheveux détectées au moyen d'au moins un capteur (1a, 1b) dans un dispositif de stockage (8) lorsqu'un premier modèle de mouvement est détecté ; et/ou
• la suppression d'au moins une information concernant l'état des cheveux stockée dans le dispositif de stockage (8) et/ou dans le dispositif de stockage externe (9) lorsqu'un deuxième modèle de mouvement est détecté ; et/ou
• la transmission des informations concernant l'état des cheveux à un dispositif de traitement de données externe (10) et/ou à un dispositif de stockage externe (9) lorsqu'un troisième modèle de mouvement est détecté ; et/ou
• l'affichage d'informations sur la base des informations concernant l'état des cheveux sur un dispositif d'affichage (11) du dispositif et/ou sur un dispositif d'affichage (12) du dispositif de traitement de données externe (10) lorsqu'un quatrième modèle de mouvement est détecté.

15. Procédé selon l'une des revendications 13 ou 14, dans lequel le procédé comprend en outre :
• la réalisation de la détection de la couleur des cheveux dans un premier temps ; et
• la réalisation de la détection du degré d'endommagement des cheveux dans un second temps, le premier temps et le second temps se situant dans une plage de temps prédéfinie.
